# EUROPÄISCHE PATENTSCHRIFT

(11) **EP 0 616 639 B1**
(45) Veröffentlichungstag und Bekanntmachung des Hinweises auf die Patenterteilung: **04.11.1998**
(21) Anmeldenummer: 92924637.9
(22) Anmeldetag: 01.12.1992
(51) Int. Cl.: C12N 1/08, C12N 15/10

(54) **VORRICHTUNG UND VERFAHREN ZUR ISOLIERUNG UND REINIGUNG VON NUKLEINSÄUREN**
DEVICE AND PROCESS FOR ISOLATING AND PURIFYING NUCLEIC ACIDS
DISPOSITIF ET PROCEDE POUR L'ISOLATION ET LA PURIFICATION D'ACIDES NUCLEIQUES

(30) Priorität: 02.12.1991 DE 4139664
(43) Veröffentlichungstag der Anmeldung: 28.09.1994
(60) Teilanmeldung: 98107576.5
(73) Patentinhaber: QIAGEN GmbH, 40724 Hilden (DE)
(72) Erfinder: COLPAN, Metin, D-4300 Essen (DE)
(74) Vertreter: Meyers, Hans-Wilhelm, Dr.Dipl.-Chem.
(86) Internationale Anmeldenummer: EP9202775
(87) Internationale Veröffentlichungsnummer: WO9311221

(56) Entgegenhaltungen:
- EP-A- 0 268 946
- EP-A- 0 376 080
- EP-A- 0 389 063
- WO-A-91/05606
- WO-A-91/07422
- US-A- 4 810 381
- US-A- 4 935 142
- US-A- 5 075 430

## Beschreibung

Die Erfindung betrifft ein Verfahren zur Isolierung und Reinigung von Nukleinsäuren wie Plasmid- oder genomischer DNA aus Zellen oder anderen Quellen und eine Vorrichtung zur Durchführung des Verfahrens gemäß Oberbegriff des Patentanspruchs 16.

Bei der Präparation von Nukleinsäuren müssen die Zellen zunächst durch die Verwendung von Enzymen, wie zum Beispiel Proteinase K, Lysozym und Detergentien wie SDS, Brij, Triton-X-100, Tween 20,DOC und Chemikalien wie Natriumhydroxid, Guanidin-Hydrochlorid und Guanidin-Isothiocyanat aufgeschlossen werden. Dem Experimentator stellt sich das Problem, vor der Reinigung der Nukleinsäuren die Zelltrümmer zu entfernen und dann aus dem Zell-Lysat die Nukleinsäuren oder Nukleinsäurefraktionen zu isolieren. Weiterhin müssen bei der Präparation von Plasmid DNA oder genomischer DNA häufig verwendete Detergentien, wie SDS (Sodiumdodecylsulfat), entfernt werden. Dies erfolgt wie in den meisten Fällen bei Verwendung von SDS durch ein Ausfällen mit Kalziumacetat, da das Kaliumsalz von SDS schwer löslich ist. Die Zelltrümmer werden dann zusammen mit dem ausgefallenen SDS abzentrifugiert. Da die Bestandteile im Lysat ein sehr voluminöses und schmieriges, gelartiges Pellet ergeben, bereitet selbst die Abtrennung dieser Trümmer in einer hochtourigen Zentrifuge Schwierigkeiten. Üblicherweise erfolgt die Entfernung der Zelltrümmer durch eine Zentrifugation zwischen 5.000 g bis 20.000 g für 15 bis 60 Minuten. Dieses Verfahren hat den Nachteil, daß es sehr zeit- und arbeitsaufwendig ist und sich nicht automatisieren läßt.

Die DE-A 36 39 949 beschreibt ein Verfahren zur Isolierung und Reinigung langkettiger Nukleinsäuren von anderen Substanzen aus Bakterien, Viren, tierischen und pflanzlichen Geweben und Zellen sowie Körperflüssigkeiten, insbesondere Zellinhaltsstoffen und/oder deren Abbauprodukten sowie Bestandteilen der Körperflüssigkeiten, die nicht langkettige Nukleinsäuren sind. Dabei werden die langkettigen Nukleinsäuren nach einem schonenden Aufschluß und Entfernung der Zellbruchstücke und anderer ungelöster Bestandteile an einem Anionenaustauscher fixiert, während die abzutrennenden Substanzen ausgewaschen werden. Danach werden die fixierten Nukleinsäuren mit einem Puffer hoher Ionenstärke von der Matrix wieder abgelöst.

Aus der DE-A 37 17 211 ist ein Verfahren bekannt zur Trennung und Reinigung von Biopolymeren, wie Nukleinsäuren, wobei die Nukleinsäuren an einer in einer speziellen Vorrichtung angeordneten Matrix adsorbiert werden. Die Pufferbedingungen sind dabei so eingestellt, daß die Nukleinsäuren überwiegend adsorbiert werden, während störende Substanzen, wie Proteine, niedermolekulare Stoffe oder auch Zelltrümmer, nicht gebunden werden.

Die EP 0 376 080 beschreibt ein Verfahren zur Extraktion und Reinigung von DNA, ausgehend von Lambda-Phagen, M13 Phagemiden, Plasmiden, Kosmiden etc., wobei das Verfahren mehrere Filtrationsschritte umfaßt.

Die US 5,075,430 offenbart ein Verfahren zur Reinigung von Plasmid- und anderer DNA (einzel- und doppelsträngig) durch Immobilisierung auf Diatomeenerde in Anwesenheit eines chaotropischen Agens und anschließender Elution der DNA mit Wasser oder einem schwach konzentrierten Puffer. Die so erhaltene gereinigte DNA ist biologisch aktiv.

Die WO 91/05606 betrifft eine chromatographisches Trägermaterial, dessen Hohlräume die 1- bis 20fache Größe der größten Abmessung der zu trennenden Nukleinsäuren aufweisen, welches dadurch erhältlich ist, daß ein Ausgangsträgermaterial einer Hohlraumgröße von 10 bis 1.000 nm, einer spezifischen Oberfläche von 5 bis 800 m²/g und einer Korngröße von 3 bis 500 µm mit einem Silanisierungsreagens umgesetzt wird, daß dadurch gekennzeichnet ist, daß das Silanisierungsreagens mindestens eine bereits mit einem primären oder sekundären Hydroxyalkylamin umgesetzte reaktive Gruppe aufweist.

Die WO 91/07422 beschreibt ein Verfahren und ein Kit zur Reinigung von Nukleinsäuren, wie z.B. DNA, aus lysierten Zellen oder Gewebeproben. Die Probe wird dann mittels Anionenaustauscher-Chromatographie gereinigt.

Die US 4,810,381 beschreibt eine Vorrichtung bestehend aus PTFE-haltiger fibriler Matrix, welche mit nicht-blähbaren Partikeln vernetzt ist. Dabei liegt das Verhältnis nichtblähbarer Partikel zu PTFE im Bereich von 19:1 bis 4:1 Gew.-%.

Die US 4,935,142 offenbart eine Membraneinheit bestehend aus einer variablen Anzahl von gestapelten, planaren Membranelementen als Filter für die Chromatographie.

Die EP 0 890 063 beschreibt ein Verfahren und ein Testkit zur Isolierung von Nukleinsäuren aus nukleinsäurehaltigen Material wie Blut, Urin, Zellkulturen und ähnliches.

In "Isolierung, Fraktionierung und Hybridisierung von Nukleinsäuren, eine Einführung und methodische Anleitung", herausgegeben von Ulrich Wobus, Verlag Chemie, 1980 werden Methoden zur Isolierung von Nukleinsäuren beschrieben. Daraus geht hervor, daß hochmolekulare Ribonukleinsäuren in Salzlösungen > 1,5 M Natriumchlorid unlöslich sind und ausfallen. Diese Präzipitation wird jedoch als nicht effizient angesehen, so daß in dieser Monographie bereits mehrfache Wiederholungen der Präzipitationsschritte mit hoher Salzkonzentration empfohlen werden.

Eine effiziente Trennung sowohl von DNA-Restriktionsfragmenten und amplifizierten Produkten der Polymerase-Kettenreaktion wird in J. Chromatogr., 1990, 512, 433 - 444 beschrieben. Als Chromatographiematerial wird ein Ionenaustauscher DEAD-NPR-Material mit 2,5 µm großen, nicht porösen Partikeln verwendet.

Nukleinsäure aus Hefen wurden gemäß Biochemistry 1972, 4848 an Poly(L-Lysine)-beschichtetem Kieselguhr getrennt. Ebenso wurde bereits mitochondriale DNA an solchen chromatographischen Materialien getrennt.

In Chromatographia, 1984, 19, 236 - 9 wird die Verwendung von mehrdimensionaler Chromatographie zur Isolierung von synthetischen Oligodeoxyribonukleotiden im präparativen Maßstab beschrieben. In einem ersten Schritt wird dabei zunächst eine Size-Exclusion-Chromatographie an Sephadex G-15 durchgeführt, gefolgt von einer Size-Exclusion-Chromatographie mit einer HPLC-Ionenaustauschersäule (Partisil-10 SAX). Daran schließt sich eine hydrophobe Chromatographie mittels HPLC (Nucleosil C18) an.

Über die Eignung von hydrophob beschichteten Glaspartikeln zur Durchführung von adsorptions-chromatographischer Reinigung von Nukleinsäuren wird in J. Biochem. 94, 163 - 169 (1983) berichtet.

Nachteilig an diesem stellvertretenden Stand der Technik ist die Tatsache, daß ein Zentrifugationsschritt zur Entfernung der Zellbruchstücke und der ungelösten Bestandteile aus dem Zell-Lysat notwendig ist. Ein weiteres Problem besteht darin, daß die Nukleinsäuren durch die Elution in Puffern hoher Ionenstärke von den in großer Konzentration vorhandenen Salzen befreit und gleichzeitig konzentriert werden müssen. In den allermeisten Fällen sind die weiteren Verfahrensoperationen mit den so gewonnenen Nukleinsäuren nur mit Pufferbedingungen möglich, die geringere Ionenstärken aufweisen. Die Entfernung der in hoher Konzentration im Puffer gelösten Salze kann auch durch Dialyse erfolgen, jedoch führt dies zu merklicher Degradation der Nukleinsäuren in den entsprechenden Proben. Nach der Dialyse muß die entsalzte Nukleinsäure durch eine Gefriertrocknung konzentriert werden. Eine andere Art der Konzentrierung erfolgt durch eine Fällung der Nukleinsäure mit Ethanol, Isopropanol, Polyethylenglykol (PEG). Die Nukleinsäuren sind in diesem System nicht löslich und fallen aus. Die ausgefallenen Nukleinsäuren müssen jedoch durch einen Zentrifugationsschritt pelletiert werden. Das Nukleinsäurepellet wird kurz getrocknet und anschließend in einem kleinen Volumenpuffer sehr niedriger Salzkonzentrationen gelöst, um eine konzentrierte salzfreie Nukleinsäureprobe zu erhalten. Durch diese Zentrifugations- und Fällungsverfahren ist eine einfache und schnelle Gewinnung von Nukleinsäuren nicht möglich und eine Automatisierung läßt sich nur schwer durchführen. Andererseits steigt der Bedarf nach einfachen und automatischen Verfahren zur Präparation von Nukleinsäuren durch das Vordringen der Molekularbiologie in die klinische Diagnostik sowie die Sequenzierung des menschlichen Genoms. Dabei sind jeweils große Probenmengen aufzuarbeiten.

Das der Erfindung zugrundeliegende technische Problem besteht darin, ein Verfahren bereitzustellen, daß es ermöglicht, Nukleinsäuren zu isolieren und zu reinigen, ohne daß ein Zentrifugationsschrift zur Entfernung der Zellbruchstücke oder ungelöster Bestandteile des Zell-Lysats notwendig wäre und, ohne daß die Nukleinsäuren in Puffersystemen hoher Salzkonzentrationen anfallen, wobei die Nukleinsäuren einen nachgeschalteten Entsalzungs- und Konzentrierungsschritt notwendig machen. Das bereitzustellende Verfahren soll die Nukleinsäuren praktisch in einem direkt weiterverarbeitbaren Zustand liefern. Ein weiterer Aspekt des genannten technischen Problems besteht in der Schaffung einer Vorrichtung, mit der das Verfahren in besonders vorteilhafter Weise ausgeführt werden kann.

Das der Erfindung zugrundeliegende technische Problem wird in überraschend einfacher Weise durch ein Verfahren gelöst, daß durch die Merkmale des Anspruchs 1, 34, 36 charakterisiert ist. Die daran anschließenden Verfahrensansprüche betreffen bevorzugte Ausführungsformen des erfindungsgemäßen Verfahrens.

Eine Vorrichtung, mit der das erfindungsgemäße Verfahren in besonders vorteilhafter Weise ausgeführt werden kann, ist durch die Merkmale des Anspruchs 16, 35, 37 charakterisiert. Die darauf zurückbezogenen Unteransprüche betreffen weitere bevorzugte Ausführungsformen der erfindungsgemäßen Vorrichtung.

Zunächst werden die Zellen, deren Nukleinsäure isoliert werden sollen, in üblicher Weise aufgeschlossen und die Zelltrümmer werden entfernt. Dies kann mittels Filtration oder Zentrifugation geschehen. Vorzugsweise erfolgt die Gewinnung der klaren Zell-Lysate durch eine Filtration über eine stufenweise oder asymetrisch aufgebaute Filterschicht. Das die Nukleinsäuren enthaltende Filtrat kann sofort mit Anionenaustauschern behandelt werden. Als Anionenaustauscher kann ein handelsübliches Material ausgewählt werden, welches eine Bindung der zu isolierenden Nukleinsäure unter den jeweiligen Präparationsbedingungen erlaubt. Die Anionenaustauscher sind vorzugsweise oberflächenmodifizierte Träger aus einer Matrix, vorzugsweise bestehend aus Agarose, Dextran, Zellulose, Acrylamid, Polyvinylalkohol, Polystyrol, Glas, Aluminiumoxid, Titandioxid, Zirkondioxid oder Silicagel, wie zum Beispiel DEAE-Sepharose^{R}, Q-sepharose^{R}, DEAE-sephadex^{R}, DEAE-Toyopearl^{R}, Amberlite^{R}, Nukleogen^{R}, Qiagen^{R}. Die Anionenaustauscher können poröse Trägermaterialien mit einer zur Wechselwirkung geeigneten inneren Oberfläche hoher Kapazität oder nicht poröse Trägermaterialien sein, die nur auf der äußeren Oberfläche eine Wechselwirkung mit dem zu trennenden Gemisch eingeht. Ganz besonders bevorzugt handelt es sich bei dem Anionenaustauscher um ein Material auf Basis von Silicagel, das eine Partikelgröße von 1 bis 250 µm, vorzugsweise 10 bis 50 µm und ganz besonders bevorzugt 15 bis 25 µm und einen Porendurchmesser von 1 bis 2.500 nm, bevorzugt 10 bis 500 nm, besonders bevorzugt 100 bis 400 nm, aufweist. Als Anionenaustauschermaterial hat sich insbesondere ein Material mit hoher Oberflächenladung und hoher Bindungskapazität für Nukleinsäuren erwiesen. Die Modifizierung des Silicagels erfolgt vorzugsweise durch Silanisierung des Trägermaterials, wie beispielsweise in der EP-A 83 901 065, DE-A-39 35 098 und US-A-5,057,426 offenbart. In der EP-A 83 901 065 wird zum Beispiel gamma-Glycidyloxypropyltrimethoxysilan und N,N-Dimethylaminoethanol zur Modifizierung des Trägermaterials verwendet.

Die Adsorption der Nukleinsäuren erfolgt unter Bedingungen, wie sie typischerweise bei niedrigen Salzkonzentrationen vorliegen. Vorzugsweise sind dies niedrigere Salzkonzentrationen als solche mit der die Nukleinsäuren von der Säule eluiert werden können. Je nach verwendeten Ionenaustauschermaterialien und pH-Werten kann die Salzkonzentration dabei 0,25 bis 1,5 M betragen.

Nach der Adsorption der Nukleinsäuren an dem Anionenaustauschermaterial kann sich mindestens ein Waschschritt mit Puffer geringer Ionenstärke anschließen.

Vorzugsweise befindet sich das Ionenaustauschermaterial dabei in einem überwiegend zylindrischen Hohlkörper einer Säule. Die Säule wird dann mit einer Salzlösung gewaschen, deren Ionenstärke so hoch wie möglich ist, ohne daß die erwünschte Nukleinsäure eluiert wird. Damit werden niedermolekulare und schwach geladene Verunreinigungen und Proteine ausgewaschen.

Um unnötige Ausbeuteverluste zu vermeiden, kann es vorteilhaft sein, zwischen dem Adsorptionsschritt und dem Ellutionsschritt oder als letzten Waschschritt eine Konditionierung der betreffenden Adsorptionsmaterialien durchzuführen, indem eine möglichst hohe Ionenstärke insbesondere in dem Bereich, in dem die spätere Adsorption der Nukleinsäure unter Hochsalzbedingungen erfolgen soll, durchgeführt wird. Dazu kann insbesondere eine Lösung zur Äquilibrierung und Konditionierung verwendet werden, die einer Ionenstärke von etwa 1,5 M Natriumperchlorat bei einem pH von ungefähr 5 entspricht.

Entsprechend befindet sich das Material zur Bindung der Nukleinsäuren unter Bedingungen hoher Ionenstärke in einem separaten überwiegend zylindrischen Hohlkörper. Die von dem Ionenaustauschermaterial desorbierte Nukleinsäurefraktion wird in der hoch salzhaltigen Fraktion in die Kartusche oder die Säule mit dem Nukleinsäuren unter Hochsalzbedingungen absorbierenden Material gegeben. In einer bevorzugten Ausführungsform sind die jeweiligen Vorrichtung mit den entsprechenden Adsorptionsmaterialien so aufeinander abgestimmt, daß der Behälter mit dem Anionenaustauscher auf den Behälter mit dem Nukleinsäure unter hohen Ionenstärken bindenden Material enthaltenden Behälter angeordnet werden kann.

Eine Konditionierung des Materials, das die Nukleinsäuren bei hoher Ionenstärke adsorbieren kann, ist insbesondere bei dieser Vorgehensweise besonders leicht möglich. Die Konditionierung kann bereits dadurch erfolgen, daß das Material, das die Nukleinsäuren bei hoher Ionenstärke binden kann, mit entsprechend hochkonzentrierten Salzlösungen vorbehandelt wird. Es ist jedoch auch möglich, entsprechend vorbehandelte Materialien zu verwenden, indem beispielsweise das Nukleinsäure absorbierende Material zunächst mit Salzlösungen hoher Ionenstärke behandelt wird und danach das Lösungsmittel verdampft wird, so daß sich in den Nukleinsäuren unter hohen Ionenstärken absorbierende Material sehr hohe Salzkonzentrationen unmittelbar einstellen, wenn eine wäßrige Lösung damit in Verbindung gebracht wird. Vorteilhaft ist die Konditionierung aufgrund der Tatsache, daß die ersten Volumeneinheiten, die sich durch die Elution der Nukleinsäuren vom Anionenaustauscher von diesem Material lösen noch eine möglicherweise zu geringe Salzkonzentration aufweisen, um hinreichend fest an dem folgenden Material zu adsorbieren. Trifft nun ein relativ verdünnter Elutionstropfen vom Anionenaustauscher auf ein so konditioniertes Material, das Nukleinsäure unter Bedingungen hoher Ionenstärke zu binden vermag, so stellt sich sofort eine hohe Salzkonzentration ein und die Nukleinsäuren werden an diesem Material adsorbiert.

Danach kann die Nukleinsäure mit einem Puffer hoher Ionenstärke von dem Anionenaustauschermaterial desorbiert, um dann unmittelbar im Elutionspuffer hoher Ionenstärke mit einem mineralischen Träger gebunden zu werden. Nukleinsäuren können in Gegenwart von chaotropen Salzen wie Natriumiodid, Natriumperchlorat an feingemahlenem Glas oder Silicagel gebunden werden, wenn man die Nukleinsäuren mit der feinen Glas- bzw. Silicagelsuspension versetzt und längere Zeit inkubiert, um eine Bindung der Nukleinsäure an das Silicagel zu ermöglichen (B. Vogelstein und D. Gillespie, 1979, Proc. Nat. Aca. Sci USA, 76, 615 - 19; Preparative and analytical purification of DNA from agarose; R. Yang, J. Lis und B. Wu, 1979, Elution of DNA from agarose after gel electrophoresis, Methods Enzymol. 65, 176 - 182; M.A. Marko, R. Chipperfield und H.C. Birnboim, 1982, A procedure for the large scale isolation of highly purified plasmid DNA using alkaline extraction and binding to glass powder, Anal. Biochem, 121, 382 - 387).

Die Adsorption der Nukleinsäuren an die mineralischen Träger kann überraschen derweise auch durch Zugabe von niederen Alkoholen in die Probe erfolgen. In Frage kommen vorzugsweise Methanol, Ethanol, Propanol, Isopropanol sowie Butanol. Die bevorzugten Mengenbereiche, in denen die Alkohole der Probe zugesetzt werden, betragen 1 - 50% (v/v), soweit sie überhaupt in diesen Bereichen in Wasser löslich sind. Desweiteren kann die Adsorption der Nukleinsäuren auch durch Polyethylenglykole erreicht werden. Die verwendbaren Ethylenglykole weisen Molekulargewichte von 1.000 bis 100.000, insbesondere 6.000 bis 8.000, auf. Polyethylenglykol kann in Bereichen von 1 - 30% der Probe zugesetzt werden.

Das erfindungsgemäße Verfahren zeigt überraschenderweise, daß Nukleinsäure auch beim Passieren von sehr dünnen Schichten von Glas oder Silicagel effizient adsorbieren, obwohl die Verweilzeit nur 1 - 30 Sekunden beträgt. Es zeigt sich auch, daß eine Bindung in hohen Natriumchlorid- und Lithiumchloridkonzentrationen erfolgt und chaotrope Salze nicht notwendig sind. Auch ist bisher eine Kombination aus Anionenaustauscher und Silicagel nicht beschrieben, wobei der Anionenaustauscher die Reinigung der Nukleinsäure übernimmt und bei den Konzentrationen von 0,25 M - 1,5 M Salz zwar die Verunreinigungen, wie Metaboliten, Proteine und teilweise RNA, Polysaccharide entfernt werden, aber diese unter den gegebenen Bedingungen nicht an die nachgeschaltete Silicagelschicht adsorbieren können, und die Silicagelschicht die Entsalzungs- und Konzentrationsaufgabe übernimmt, wenn die Nukleinsäure im folgenden Schritt mit einer Salzkonzentration vom Anionenaustauscher eluiert wird, die hoch genug ist die Nukleinsäure an die Silicagelschicht adsorbieren kann.

Als Puffersalze in den angegebenen Konzentrationen kommen für den Adsorptionsschritt an den mineralischen Träger folgende in Betracht:

| Salz | Konzentration |
|---|---|
| NaCl | 3 - 5 M |
| NaClO4 | 5 - 7 M |
| Gu-HCl | 5 - 7 M |
| NaJ | 3 - 5 M |

Die Behandlung mit der Salzlösung kann einfach durch Auftropfen auf den Filter und Absaugen erfolgen. In einer bevorzugten Ausführungsform wird die Silicagelschicht mit einer Perchloratlösung, pH 6,5 bis 8,5, insbesondere pH 7 bis 8, behandelt. Dies erfolgt zweckmäßig durch Pipettieren und Durchsaugen. Besonders bevorzugt wird hierzu eine Lösung, die 4 bis 8 M/1 NaClO4, 5 bis 20 mM/l Tris-HCl, pH 7 bis 8 und 0,5 bis 2 mM/l EDTA enthält, verwendet. Nach dem Entfernen der chaotropen Lösungen, insbesondere der Natriumperchloratlösung, wird vorzugsweise mit wäßrigem Ethanol nachgewaschen, zum Beispiel mit 50 bis 90%-igem Ethanol.

Nach dem Trocknen der Filter erfolgt dann die Elution in üblicher Weise mit einer verdünnten wäßrigen Salzlösung, wie z. B. in Anal. Biochem. 101, 339 - 341 (1980) beschrieben. Ein bevorzugtes Elutionsmittel ist 0,5 bis 2 mM/l Tris-HCl, pH 7 bis 8, enthaltend 0,05 bis 0,2 mM/l EDTA, im folgenden als TE bezeichnet. Besonders bevorzugt wird ein pH-Wert von 7,5 bis 8,5. Ein anderes geeignetes Elutionsmittel sind verdünnte Detergenslösungen, wie zum Beispiel 0,1% SDS, die jedoch weniger bevorzugt werden.

Es hat sich gezeigt, daß außer Silicagel auch andere mineralische Träger zur Adsorption der Nukleinsäure geeignet sind. In einer bevorzugten Form wird jedoch Silicagel der Partikelgröße 1 bis 250 µm, bevorzugt 1 bis 50 µm, insbesondere 1 - 5 µm, eingesetzt. Die Entsalzungsschicht kann als eine lose geschüttete Schicht, die zwischen zwei PE-Fritten eingeschlossen ist, in der Extraktionssäule eingesetzt werden. Eine andere Ausführungsform beinhaltet die Anwendung der mineralischen Träger in Membranform nach EP 0 323 055 (07.12.1988, 3M, Composition Chromatographic Article).

Mit dem erfindungsgemäßen Verfahren können Nukleinsäuren verschiedenster Provenienz getrennt und präpariert werden. Dabei ist es gleichgültig, ob die Nukleinsäuren aus Bakterien, Zellkulturen, Blut, Gewebe, Urin, Viren oder aus Amplifikationsreaktionen, wie PCR (Polymerase Chain Reaction), SSSR (Self-Sustained-Sequence Replication), Ligase-Chain-Reaction und ähnlichen Reaktionen stammen, oder ob es sich um markierte Nukleinsäuren, wie in Biotin markierte, fluoreszens-markierte oder radioaktiv markierte Nukleinsäuren handelt. Als Nukleinsäure kommen Nukleinsäuren in einem Größenbereich vom 10 Nukleotiden bis 200.000 Nukleotiden in Betracht. Als Nukleinsäuren im Sinne der Erfindung werden Olegonukleotide von 10 bis 100 Nukleotiden, RNA mit 50 bis 25.000 Nukleotiden, Plasmid-DNA mit 2.500 bis 25.000 Basenpaaren, Cosmid-DNA mit 5.000 bis 60.000 Basenpaaren oder genomische DNA mit 100 bis 200.000 Basenpaaren verstanden.

Die nach Schritt d) des erfindungsgemäßen Verfahrens erhaltene Nukleinsäurefraktion oder -fraktionen werden in Lösungen mit geringer Salzbelastung erhalten. Es ist somit möglich, die für die weitere Prozessierung erforderlichen Pufferbedingungen nachträglich einzustellen. In besonders vorteilhafter Weise wird die an dem Silicaglas gebundene Nukleinsäure bereits in dem zur Weiterverarbeitung bestimmten Puffer eluiert.

Die isolierten Nukleinsäuren werden für die unterschiedlichsten Anwendungen eingesetzt. Besonders häufig erfolgt die enzymatische Umsetzung mit Restriktionsenzymen, Polymerasen und Ligasen zur Restriktionsanalyse, Sequenzierung, Markierung mit Radioaktivität oder nicht radioaktiven Markern, wie Biotin, FITC, Digoxigenin und der Amplifikation mit Hilfe der PCR, SSSR (Self-Sustained-Sequence Replication) und Ligase-Chain-Reaction.

Das erfindungsgemäße Verfahren ist insbesondere für die Isolierung und Präparation von Plasmid-DNA und genomischer DNA geeignet.

Die Figuren zeigen bevorzugte Ausführungsformen der erfindungsgemäßen Vorrichtung, wobei die verschiedenen Adsorptionsmaterialien für die Nukleinsäuren in einer Vorrichtung vereint sind.

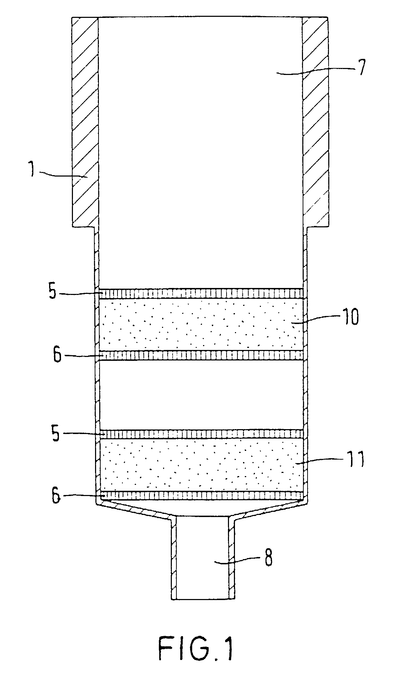

Die Figur 1 zeigt eine Vorrichtung zur Durchführung des erfindungsgemäßen Verfahrens, die aus einem Hohlkörper 1 mit einer Einlaßöffnung 7 und einer Auslaßöffnung 8 besteht. Der Hohlkörper besteht vorzugsweise aus Polypropylen (PP), Polyethylen (PE), Polymethylmethacrylat (PMMA), Polytetrafluorethylen (PTEE), Polyethylterephthalat (PET) oder Polyacrylnitril (PAN). Im Hohlkörper 1 ist zwischen zwei Fixiereinrichtungen 5, 6 ein pulverförmiges erstes Material aus einem mineralischen Trägermaterial 10 angeordnet. Im Hohlkörper 1 befindet sich ein zweites pulverförmiges Material 11 aus einem mineralischen Trägermaterial zwischen dem ersten Material 10 und der Auslaßöffnung 8. Die ersten und zweiten Materialien 10, 11 weisen unterschiedliche Adsorptionscharakteristika für Nukleinsäuren auf. Die Unterschiede in den Adsorptionscharakteristika werden durch unterschiedliches Adsorptionsverhalten in Puffern hoher bzw. niedriger Ionenstärken bestimmt. Werden zum Beispiel Nukleinsäuren vom ersten Material 10 unter Bedingungen niedriger Ionenstärke gebunden, so muß das zweite Material 11 in der Lage sein, Nukleinsäuren unter Pufferbedingungen niedriger Ionenstärke ungehindert passieren zu lassen, wohingegen unter Bedingungen hoher Ionenstärke die Nukleinsäure vom ersten Material 10 desorbiert und an dem zweiten Material 11 adsorbiert wird.

Vorzugsweise besteht das erste pulverförmige Material 10 aus einem Anionenaustauscher aus oberflächenmodifizierten Trägermaterialien auf Basis von Agarose, Dextranen, Cellulose, Acrylamid, Polyvinylalkohol, Polystyrol, Glas, Aluminiumoxid, Titanoxid, Zirkondioxid oder Silicagel, insbesondere Anionenaustauscher der oben genannten Art auf Silicagelbasis. Der vorzugsweise basische Ionenaustauscher weist eine Partikelgröße von 1 bis 250 µm, bevorzugt von 10 bis 40 µm, insbesondere 15 bis 25 µm, und einem Porendurchmesser von 1 bis 2.500 nm, vorzugsweise 10 bis 500 nm, insbesondere 200 - 400 nm, auf.

Das zweite Material 11 ist ein mineralisches Trägermaterial, insbesondere aus Silicagel, Glas, Zeolith, Aluminiumoxid, Titandioxid, Zirkiondioxid, Kaolin, Kieselalgen, vorzugsweise ein Silicaglas, gegebenenfalls in Form einer Silicagelsuspension. Das zweite Material 11 weist vorzugsweise eine Partikelgröße von 1 - 250 µm, insbesondere 1 bis 30 µm, bevorzugt 1 bis 5 µm, auf.

Die Einrichtungen 5 und 6 bestehen vorzugsweise aus gesintertem Glas (Fritten) oder Membranen aus Kunststoff, wie Polyethylen, PTFE, Polypropylen, Glas, Keramik, Nylon oder ein Vlies aus Polypropylen, Poylethylen, Nylon. Die Porosität der Einrichtungen 5, 6 beträgt vorzugsweise 10 bis 500 µm.

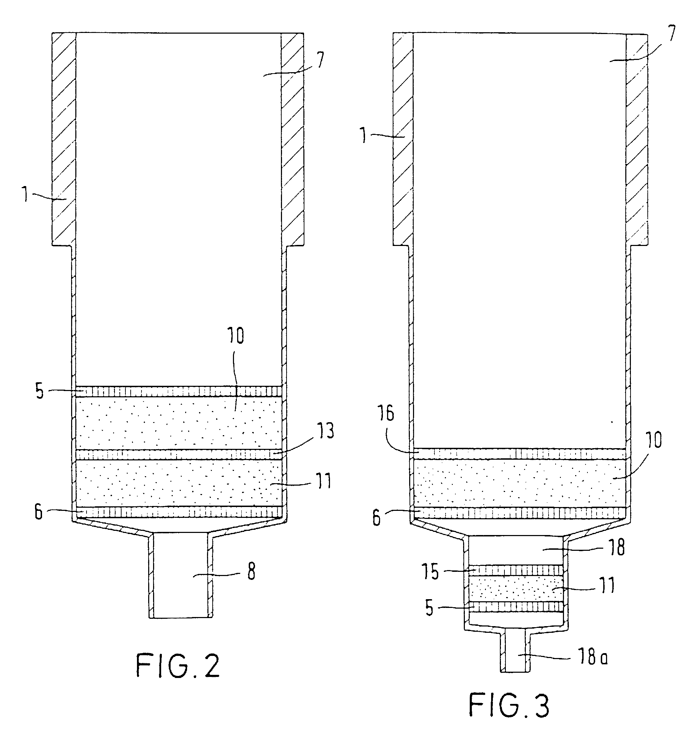

Eine weitere bevorzugte Ausführungsform der erfindungsgemäßen Vorrichtung zeigt die Figur 2. Dort ist das erste Material 10 und das zweite Material 11 so im Hohlkörper 1 angeordnet, daß die Materialien 10, 11 direkt aneinandergrenzen und zwar in getrennten Schichten, die gemeinsam von den Fixiereinrichtungen 5, 6 gehalten werden. Vorzugsweise kann das Material durch eine Trenneinrichtung 13 getrennt werden, wobei die Trenneinrichtung 13 eine poröse Scheibe, vorzugsweise aus gesintertem Glas, oder eine Kunststoffmembran, oder Gewebe, vorzugsweise aus Nylon, ist.

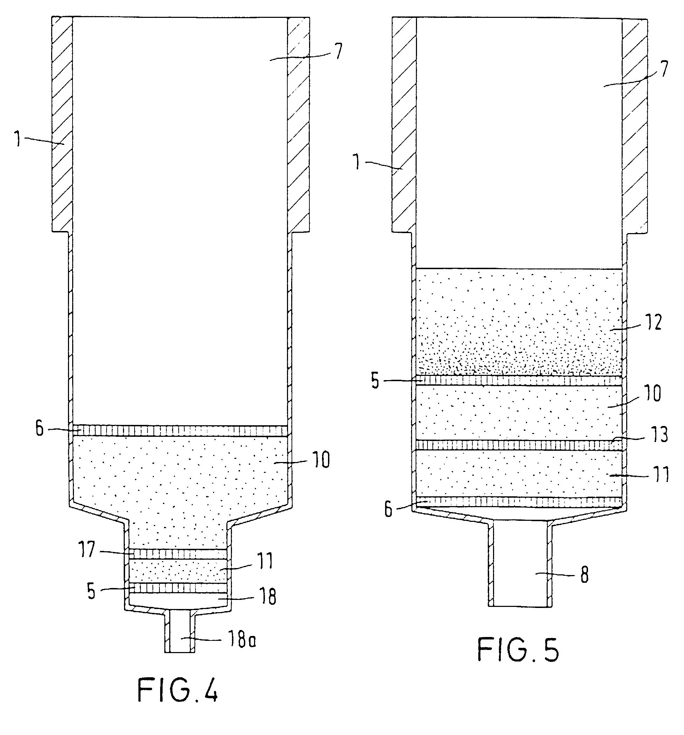

Die Figur 3 zeigt eine weitere bevorzugte Ausführungsform der erfindungsgemäßen Vorrichtung, wobei das zweite Material 11 in dem einen Kanal bildenden Auslaß 18 zwischen den Fixiereinrichtungen 5, 15 fixiert ist. Die einen Kanal bildende Auslaßöffnung 18 weist einen geringeren Querschnitt als der Hohlkörper 1 auf und mündet vorzugsweise in einem Kanal 18a, dessen Querschnitt geringer als derjenige des Kanals 18 ist. Das erste Material 10 befindet sich im Lumen des Hohlkörpers 1 im Bereich des größeren Durchmessers und ist durch die Einrichtung 6, 16 fixiert. Es kann dabei vorteilhaft sein, das erste und zweite Material 10, 11 aneinandergrenzen zu lassen, so daß diese nur durch eine gemeinsame Einrichtung 17 getrennt sind (siehe Figur 4).

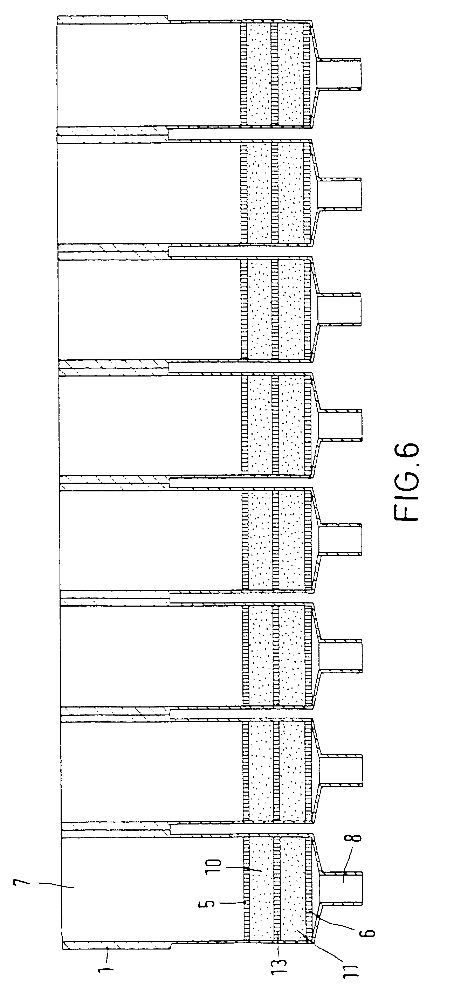

Die Figur 5 beschreibt eine weitere bevorzugte Ausführungsform der erfindungsgemäßen Vorrichtung, die im Hohlkörper neben den Schichten aus einem ersten und zweiten Material 10, 11 eine weitere Schicht 12 aufweist, die über dem ersten Material 10 angeordnet ist. Die Schicht 12 ist als mechanische Filtereinrichtung ausgebildet. Vorzugsweise ist die dritte Schicht 12 ein asymmetrischer Filter, wobei die Porengrößen des Filters in Fließrichtung der Probe, also von Zuführungsöffnung 7 zur Auslaßöffnung 8 bzw. 18, abnimmt. Damit können auch noch in der Probe befindliche Zelltrümmer entfernt werden, ohne daß die Gefahr einer Verstopfung der Vorrichtung besteht.

Die Materialien 10 und 11 können in sämtlichen Ausführungsformen der erfindungsgemäßen Vorrichtung entweder pulverförmig und/oder als Presskörper ausgebildet sein. Wenn die Materialien 10, 11 in Partikelform vorliegen, kann es empfehlenswert sein, diese in einem Trägernetz aus inerten Kunststoffen einzubetten, so daß die Schichten in Form einer Membran vorliegen gemäß US-PS 4,810,381 und US-PS 4,699,717 sowie in der DE 41 27 276 vorgeschlagen. Das Trägernetz kann aus Teflon bestehen.

Die Figur 6 beschreibt eine weitere bevorzugte Ausführungsform der erfindungsgemäßen Vorrichtung, wobei acht einmelne, getrennte Vorrichtungen gemäß Figur 2 aneinandergrenzen und eine Achtereinheit bilden. Der Vorteil dieser Ausführungsform, die mit jeder der in 1 - 5 beschriebenen Einzelformen durchführbar ist, liegt in der parallelen Präparation von 8 Proben unter Zuhilfenahme von Mehrkanalpipetten. Diese Form kann auch 12 mal aneinandergesetzt hergestellt werden, wobei 96 Proben prozessierbar werden. Der große Vorteil ist dann gegeben, wenn das international standardisierte Mikrotiter-Format verwendet wird.

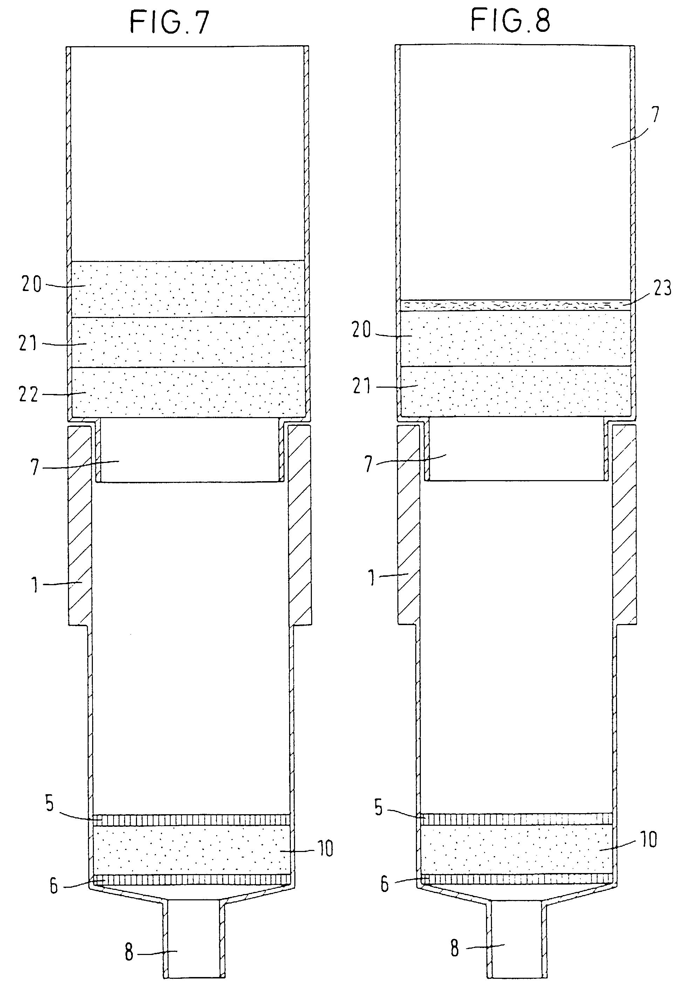

Die Figur 7 beschreibt eine Vorrichtung, die in einem zylindrischen Hohlkörper 1 mit Einlaßöffnung 7 und Auslaßöffnung 8 ein Anionenaustauschermaterial 10 zwischen zwei Einrichtungen 6 und 5 fixiert enthält. Darauf ist aufgesteckt ein weiterer zylindrischer Hohlkörper in dessen Lumen verschiedene Filterschichten angeordnet sind. Die Filterschichten 20, 21, 22 können aus gesintertem Polyethylen, Polypropylen, PTFE, Glas, Silicagel, Aluminiumoxid oder geschütteten Diatomenerde, z. B. Cellit oder Silicagel bestehen. Aber auch verwebtes, verklebtes Vlies in Form von Polypropylen, Polyester, Glasfasern und Silica kommen in Betracht. Die Porosität der einzelnen Schichten beträgt vorzugsweise 15 µm bis 500 µm in einer Dicke von 0,1 mm bis 10 mm. Die Porengröße der Filterschicht wird, in Fließrichtung gesehen, von Schicht zu Schicht geringer. In einer typischen Ausführungsform beträgt die Größe der Poren in der Schicht 20 etwa 100 bis 300 µm, in der Schicht 21 30 bis 100 µm und in der dritten Filterschicht 5 bis 30 µm.

Die Figur 8 zeigt eine weitere bevorzugte Ausführungsform der Vorrichtung nach Figur 7, wobei als, in Fließrichtung gesehen, oberste Filterschicht 23 eine hydrophobe Schicht eingesetzt wird. Die hydrophobe Trennschicht 23 verhindert die unerwünschte Penetration des rohen Zell-Lysats in die Filterschicht vor Beginn der eigentlichen Filtration. Die hydrophobe Trennschicht 23 besteht vorzugsweise aus versponnenem oder gesintertem Polypropylen, Polyethylen, Polyester oder Polytetrafluoroetylen(PTFE)-Fasern, in einer Porosität von 10 µm bis 500 µm und vorzugsweise eine Dicke von 0,1 bis 5 mm.

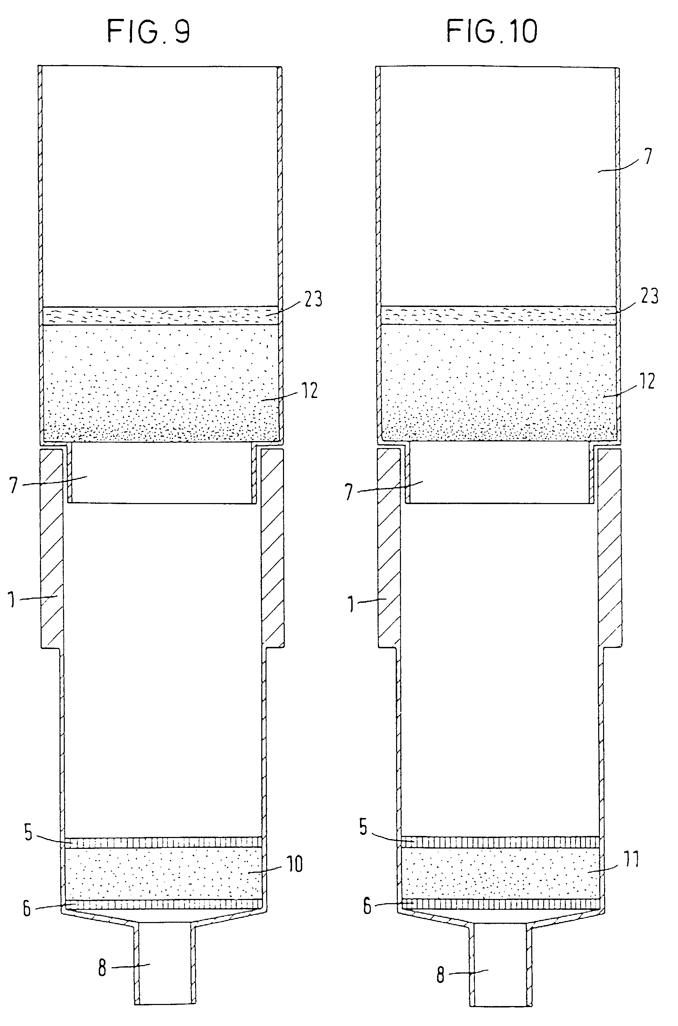

Die Figur 9 beschreibt eine Filtrationsvorrichtung, die ähnlich aufgebaut ist, wie die in den Figuren 7 und 8 beschriebenen, mit dem Unterschied, daß verschiedene Filterschichten mit abnehmender Porengröße in einer einzigen Filterschicht 12 mit kontinuierlich abnehmender Porengröße verbunden sind. Die asymmetrische Filterschicht 12 ist vorzugsweise mit einer hydrophoben Filterschicht 23 am oberen Ende, in Fließrichtung gesehen, versehen. Die asymmetrische Filterschicht 12 besteht vorzugsweise aus versponnenem Polypropylen oder Polyesterfasern; kommerziell erhältlich sind Profile, beispielsweise von Pall Filtertechnik, Dreieich, Frankfurt, mit Porositätsabstufungen von 500 bis 50 µm, 100 bis 10 µm, 50 bis 5 µm sowie 10 bis 0,1 µm. Die Dicke der asymmetrischen Filterschicht sollte vorzugsweise 1 mm bis 10 mm betragen.

Die Figur 10 beschreibt Filtrationseinrichtungen zur Abtrennung von Nukleinsäuren im erfindungsgemäßen Sinne wobei auf die Filterkonfigurationen der Figur 9 zurückgegriffen wird und wobei eine asymmetrische Filterschicht mit einer hydrophoben Filterschicht 23 versehen ist. Im Hohlkörper 1 befindet sich anstelle des Anionenaustauschers 10 ein mineralischer Träger 11, der in der Lage ist, Nukleinsäuren in hochkonzentrierten Salzlösungen zu adsorbieren.

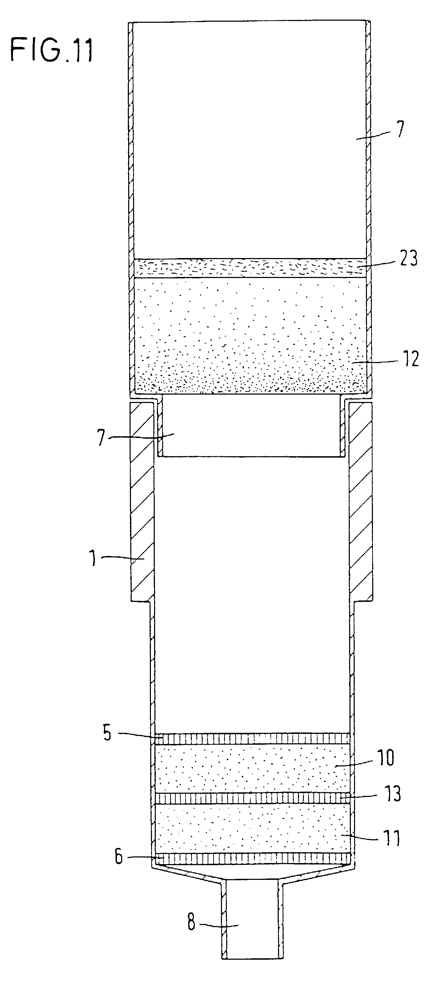

Die Figur 11 beschreibt eine Konfiguration in einer Verbindung der Figuren 9 und 10. Dabei wird der Vorrichtung, die in Figur 2 beschrieben wird, lediglich ein Filteraufsatz bestehend aus einem asymmetrischen Filter 12 und einer hydrophoben Filterschicht 23 zugeordnet etwa durch Einstecken einer entsprechend ausgebildeten Kartusche.

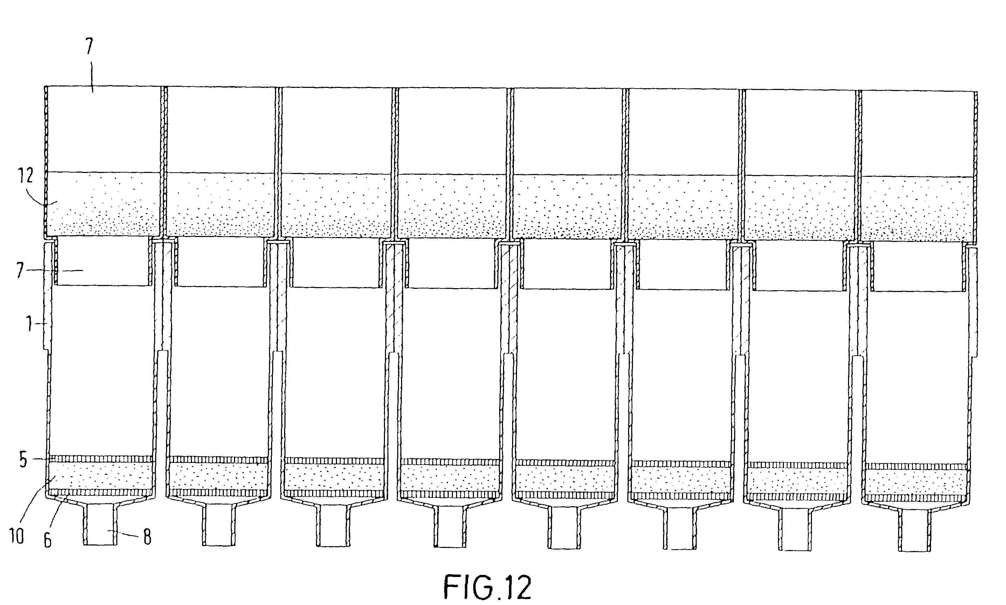

Sämtliche Einzelvorrichtungen, die in den Figuren 1 bis 5 und 7 bis 11 näher beschrieben worden sind, lassen sich in einem Mikrotiterstreifen bestehend aus 8 aneinandergesetzten Einzelvorrichtungen anordnen. Beispielhaft ist dies nocheinmal in den Figuren 12 bis 14 dargestellt.

Die Figur 12 zeigt eine Filtrationsvorrichtung mit Anionenaustauscher wobei ein Mikrotiterstrip oder eine Mikrotiterplatte mit 8 bzw. 8 x 12 Vertiefungen. In der Vorrichtung gemäß Abbildung 12 befindet sich eine asymmetrische Filtrationseinrichtung in einer aufsteckbaren Kartusche auf dem zylindrischen Hohlkörper 1, der eine Anionenaustauscherschicht zwischen den Einrichtungen 5, 6 fixiert enthält.

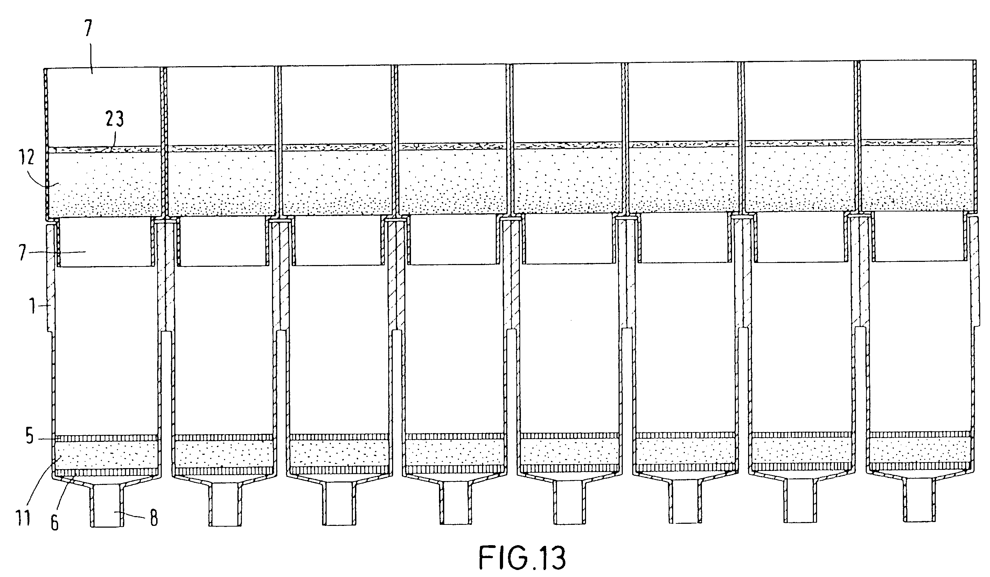

Die Figur 13 betrifft eine Filtrationsvorrichtung, die anstelle des Anionenaustauschermaterials ein mineralisches Trägermaterial besitzt, welches in der Lage ist, Nukleinsäuren in hohen Salzkonzentrationen zu adsorbieren. Vorzugsweise befindet sich eine Silicagelschicht 11 angeordnet zwischen zwei Einrichtungen 5 und 6.

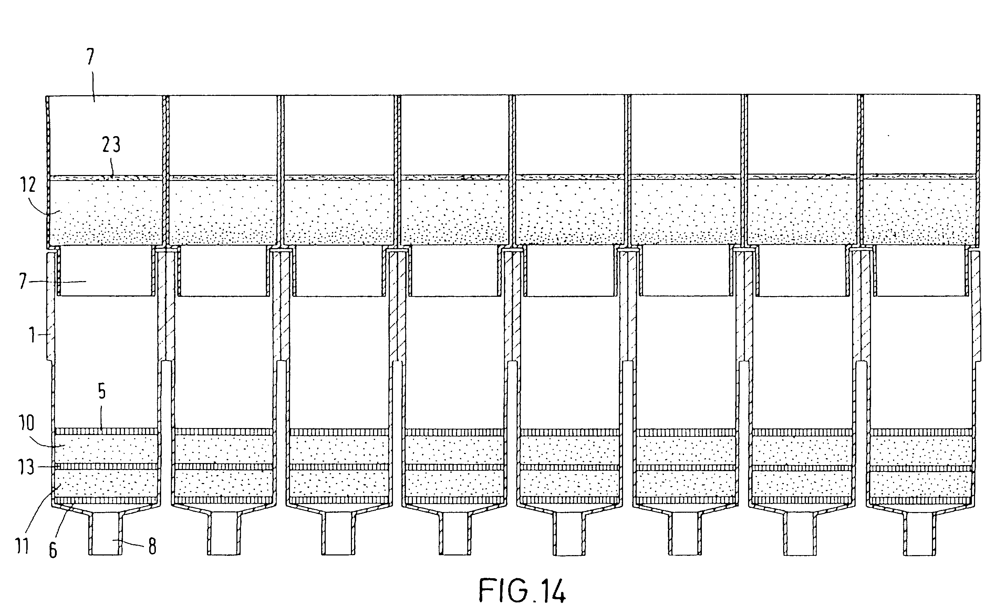

Die Figur 14 zeigt eine Kombination der Anordnung gemäß Figur 2 sowie einer asymmetrischen Filterschicht mit hydrophober Filterschicht, die über dem Hohlkörper 1, in Fließrichtung der Probe gesehen, angeordnet ist.

Die erfindungsgemäße Vorrichtung, insbesondere die in Figur 3 oder 4 näher erläuterte Vorrichtungen, sind besonders vorteilhaft, das die Elution der Nukleinsäure aus dem zweiten Material 11 mit nur sehr geringen Flüssigkeitsmengen gewährleistet.

Der Durchfluß der Probe durch die erfindungsgemäße Vorrichtung wird grundsätzlich durch die Schwerkraft bewirkt, jedoch kann zur Beschleunigung der Reinigung und Trennung der Nukleinsäuren ein Überdruck an der öffnung 7 bzw. ein Unterdruck an der öffnung 8 bzw. 18 angelegt werden. Eine weitere bevorzugte Ausführungsform der erfindungsgemäßen Vorrichtung verwendet als asymmetrische Filter solche aus gesintertem Glas mit abnehmender Porengröße oder übereinandergeschichtete Kunststoffmembranen mit abnehmender Porengröße in Fließrichtung der Probe durch den Hohlkörper.

Nukleinsäuren aus Zellen und anderen Quellen können ohne Zentrifugation, Phenol/Chloroform-Extraktion und ohne Alkoholfällung erhalten werden, wobei die Nukleinsäure am Ende des Verfahrens in konzentrierter Form in Wasser oder Puffer niedriger Salzkonzentration vorliegt und somit direkt für anschließende enzymatische Reaktionen einsetzbar ist. Ein weiterer Vorteil besteht darin, daß der Einsatz von teuren Laboreinrichtungen vermieden werden kann. Die Elution kann beispielsweise durch Schwerkraft bewirkt werden und muß nicht mittels sogenannter HPLC-Geräte durchgeführt werden.

Die Herstellung einer Silicagel-Anionenaustauscher/Silicagel-Extraktions-Säule erfolgt vorzugsweise dadurch, daß ein Polypropylen-Gefäß passend in ein handelsübliches 1,5 ml Zentrifugengefäß, unten mit einer 50 µm Polyethylen-Fritte (poröse Filterschicht aus Polyethylen, 1,5 mm dick) verschlossen wird und mit 50 mg Silicagel (Lichrosphere Si 100, 16 - 24 µm; Merck, Darmstadt, FRG) überschichtet. Diese Silicagelschicht wird mit einer zweiten porösen Polyethylen-Fritte verschlossen und die zweite Fritte mit 100 mg Silicagel-Anionenaustauscher (Qiagen, Fa. Diagen, Düsseldorf, FRG), Partikelgröße 16 bis 23 µm überschichtet und abschließend mit einer dritten porösen Polyethylen-Fritte verschlossen.

Die Herstellung einer Agarose-Anionaustauscher/Silicagel-Extraktions-Säule erfolgt vorzugsweise dadurch, daß ein Polypropylen-Gefäß unten mit einer 50 µm Polyethylen-Fritte (poröse Filterschicht aus PE; 1,5 mm dick) verschlossen und mit 50 mg Silicagel (Lichrosphere Si 100, 16 - 24 µm) überschichtet wird. Diese Silicagelschicht wird mit einer zweiten Polyethylen-Fritte verschlossen und die zweite Fritte mit 0,5 ml DEAE-Sepharose FF (Fa. Pharmacia, Freiburg, FRG), Partikelgröße 45 - 165 µm überschichtet und abschließend mit einer dritten porösen Polyethylen-Fritte verschlossen.

Die Herstellung einer Anionenaustauscher-Membrane/Silicagel-Membran-Extraktions-Säule nach Figur 3 erfolgt vorzugsweise dadurch, daß in ein Polypropylen-Gefäß auf eine Polyethylen-Fritte eine 1 mm dicke Empore^{R} Silicagelmembrane (3) (3M Corp. St. Paul, MN, USA), ein 0,2 mm dickes Polypropylen-Vlies und 1 mm dicke Anionenaustauscher-Membrane bestehen aus 16 - 23 µm Qiagen Anionenaustauscher Partikel (Diagen GmbH, Düsseldorf, FRG) plaziert wird.

Die Herstellung einer Anionenaustauscher/Silicagel-Mikrotiterstreifen-Extrations-Säule erfolgt wie beschrieben: Ein Mikrotiterstreifen mit 8 oder 96 Positionen wird mit einer DEAE.Silicagel-Membrane und einer Silicagel-Membrane gefüllt. In eine Bohrung eines Mikrotiterstreifens werden eine 0,75 mm dicke Silicagelmembrane, hergestellt aus Sident 9 Silicagelpartikeln (fa. Degussa, Frankfurt, FRG), eine 0,2 mm dicke Polypropylen-Vlies-Schicht und eine 0,8 mm dicke Anionenaustauscher-Membrane hergestellt aus Qiagen, 16 - 23 µm (Fa. Diagen, Düsseldorf, FRG) eingepaßt.

Die Erfindung wird anhand der folgenden Beispiele weiter erläutert.

### Beispiel 1

### Präparation von Plasmid DNA

Eine 100 ml Kultur in LB-Ampicillin Medium mit pUC 18 transformierten HB 101 E. coli Zellen wird 10 Minuten bei 5.000g zentrifugiert. Das Zellpellet wird in 10 ml 50 ml Tris-HCl, 10 mM EDTA, pH 8,0, 100 µg/ml RNAse A resuspendiert.

Um die Zelle zu lysieren werden 10 ml 0,2 M NaOH, 1% SDS werden zur Zellsuspension gegeben, vorsichtig gemischt und 5 Minuten bei Raumtemperatur stehen gelassen. Danach wird mit 10 ml 3M K-Acetat, 2 M Essigsäure neutralisiert, gemischt und 15 Minuten auf Eis inkubiert. Das Lysat wird 30 Minuten bei 15.000 g zentrifugiert und der Überstand vorsichtig abgehoben. 1 ml klares Zell-Lysat wird auf eine DEAE-Anionenaustauscher/Silicagel-Zentrifugations-Extraktions-Säule pipettiert und die Probe durch die Austauscherschicht 1 Minute bei 2.500 g zentrifugiert. Die Extraktionssäule wird mit 0,8 ml 1 M NaCl, 15% Ethanol, 50 mM MOPS, pH 7,0 und mit 15% Ethanol, 10 mM Na-Acetat pH 7,0, 0,8 ml 1 M NaClO₄ gewaschen, um RNA und Proteine zu entfernen. Die DNA wird mit 7 M NaClO₄, 15% Ethanol, 10 mM Na-Acetat, pH 7,0 eluiert und dabei direkt an die Silicagel-Schicht gebunden. Die Extraktionssäule wird mit 0,8 ml 70% Ethanol, 100mM NaCl, 10 mM Na-Acetat pH 7,0 und mit 0,8 ml 90% Ethanol/Wasser gewaschen. Spuren an EtOH werden eventuell durch eine weitere Zentrifugation entfernt. Anschließend wird die DNA mit 50 µl 10 mM Tris-HCl, 1 mM EDTA, pH 8,0 durch Zentrifugieren eluiert und in neuen 1,5 ml Röhrchen aufgefangen. Die eluierte DNA kann dann direkt in einer enzymatischen Reaktion wie zum Beispiel Restriktionsspaltung, Markierung, Sequenzierung oder Amplifikation eingesetzt werden.

### Beispiel 2

### Parallele Präparation von Plasmid DNA

8 DEAE-Silicagel-Membrane/Silicagel-Extraktions-Säulen werden auf einer Vakuumkammer aufgesetzt. 8 x je 1 ml eines Plasmid DNA enthaltenen Zell-Lysates werden unter Vakuum (20 bis 750 mbar) durch die Extraktionssäulen gesaugt. Die Extraktionssäule wird mit 0,8 ml 1 M NaCl, 15% Ethanol, 50 mM MOPS, pH 7,0 und mit 15% Ethanol, 10 mM Na-Acetat pH 7,0, 0,8 ml 1 M NaClO₄ gewaschen, um RNA und Proteine zu entfernen. Die DNA wird mit 7 M NaClO₄, 15% Ethanol, 10 mMNa-Acetat, pH 7,0 von der Anionenaustauscher-Schicht eluiert und dabei direkt an die Silicagel-Schicht gebunden. Die Extraktionssäule wird mit 0,8 ml 70% Ethanol, 100 mM NaCl, 10 mM Na-Acetat pH 7,0 und mit 0,8 ml 90% Ethanol/Wasser gewaschen. Die Probenröhrchen werden zur Entfernung der hochkonzentrierten Salzlösung mit 0,8 ml 70% Ethanol, 100 mM NaCl, 10 mM Na-Acetat, pH 7,0 und 0,8 ml 90% Ethanol/Wasser gewaschen. Die in der Extraktionsschicht vorhandenen Ethanol-H₂O-Reste werden durch ei nDurchsaugen von Raumluft durch ein Vakuum für 1 - 2 Minuten verflüchtigt. Anschließend werden die 8 Proben mit je 50 µl 1 mM Tris-HCl, 0,1 mM EDTA, pH 8,0 eluiert.

### Beispiel 3

### Präparation von M13 Einzelstrang DNA

1 ml M13 Phagensuspension werden mit 0,5 ml 30% PEG 6000, 1,5 M NaCl versetzt und nach 10 Minuten Inkubation auf Eis 15 Minuten bei 15.000 g abzentrifugiert. Das Phagenpellet wird in 0,5 ml 0,5 M Guanidin-HCl, 1% Triton X-100 resuspendiert und 10 Minuten bei 70°C lysiert. Das Phagenlysat wird auf einer Vakuumkammer direkt durch eine Extraktionssäule nach Beispiel 3 gesaugt und adsorbiert. Die Extraktionssäule wird mit 1 ml 0,75 M NaCl, 15% Ethanol, 50 mM MOPS, pH 7,0, lml 0,75 M NaClO₄, 50 mM Tris-HCl, pH 7,0 gewaschen und mit 7 M Guanidin, 15% Ethanol, 50 mM Na-Acetat, pH 7,0 von der Anionenaustauscherschicht eluiert und an die SiO₂-Schicht adsorbiert.

### Beispiel 4

### Präparation von genomischer DNA aus Blut

1 ml citrat-stabilisiertes, humanes Vollblut werden zur Lyse der Erythrozyten mit 1 ml 1% Saponin versetzt und sofort nach dem Mischen, 5 Minuten bei 2.500 g abzentrifugiert. Die Leukozyten werden in 1 ml PBS-Buffer resuspendiert und nochmals pelletiert. Die gewaschenen Leukozyten werden in 1 ml 500 mM Guanidin-HCl, 50 mM Tris-HCl. 10 mM EDTA, pH 8,0 resuspendiert und die Zellen durch Zugabe von 0,1 ml Proteinase K (10 mg/ml) 2 Stunden bei 50°C lysiert. Das Leukozyten-Lysat wird sofort auf die Agarose/Anionenaustauscher/Silicagel/Extraktionssäule pipettiert und mit 1 ml 0,25 M NaCl, 10 mM Na-Acetat pH 7,0 und 1 ml 0,25 NaClO₄, 10 mM Na-Acetat pH 7,0 gewaschen. 1 ml citrat-stabilisiertes, humanes Vollblut wird unter Vakuum, durch eine Anionentauscher-Silicagel-Säule gesaugt. Die Leukozyten werden dabei in der Matrix eingefangen, wogegen die wesentlich kleineren Erythrozyten durch die Matrix durchwandern. Die Extraktionssäule wird zweimal mit 1 ml PBS-Puffer nachgewaschen. Die eingefangenen Leukozyten werden mit 10 % Tween 10, 15 Minuten bei Raumtemperatur lysiert. Die Zellbruchstücke und Proteine werden mit zweimal 1 ml 1 M Guanidin-HCl, pH 7,0 ausgewaschen und die DNA mit 7M NaClO₄, 50mM Na-Acetat, pH 7,0 von der Säule eluiert.

### Beispiel 5

### Präparation, Entsalzung und Konzentration von DNA im Mikrotiterformat

96 x 1 ml Kulturen von Plasmid pBluescript in XL 1 Blue E.coli Zellen, werden im 2 x YT Medium 18 Stunden bei 37°C in einer Mikrotiterplatte mit 1,5 ml Vertiefungen (Fa. Beckmann, München) kultiviert. Die Zellen werden in einer Mikrotiterzentrifuge für 10 Minuten bei 2.500 g pelletiert. Mit einer 8-Kanal Multichanel-Pipette (Fa. Matrix Technologies, Lowell, MA, USA) werden je 0,25 ml 50 mM Tris-HCl, 10 mM EDTA, 100 µg/ml RNAglA in die Mikrotiterplattenvertiefungen pipettiert und die Zellen 5 Minuten auf einen Vibrations-Schüttler resuspendiert.

Die Zellen werden durch die Zugabe von je 0,25 ml 0,2 M NaOH, 1% SDS 5 Minuten bei Raumtemperatur unter leichtem Schütteln lysiert. Anschließend werden je 0,25 ml 3 M K-Acetat, 2 M Esigsäure, pH 5,5 - 6,0 Neutralisationspuffer zugegeben, die einzelnen Näpfe mit einer Kappe verschlossen und gemischt. Nach einer Inkubation von 10 Minute auf Eis wird die Probe 30 Minuten bei 3.000 g zentrifugiert, um die Zellbruchstücke und das präzipiterte SDS zu pelletieren. Der Überstand wir mit einer 8-Kanal-Multichanel-Pipette vorsichtig abgehoben und in die 96er Mikrotiterplatte mit einer DEAE-Silicagelmembrane und Silicagelmembrane pipettiert. Nach der Uberführung aller 96 Proben werden die Proben durch Anlegen eines Vakuums an eine Filtrierapparatur durch die Mikrotiterplatte gesaugt. Die DNA wird dabei an die Anionenaustauscher-Schicht adsorbiert, wohingegen unter diesen speziellen Bedingungen Proteine, RNA und Metabolite nicht adsorbiert werden.

Die Extraktionssäule wird mit 0,8 ml 1 M NaCl, 15% Ethanol, 50 mM MOPS, pH 7,0 und mit 15% Ethanol, 10 mM Na-Acetat pH 7,0, 0,8 ml 1 M NaClO₄ gewaschen, um RNA und Proteine zu entfernen. Die DNA wird mit 7 M NaClO₄, 15% Ethanol, 10 mM Na-Acetat, pH 7,0 eluiert und dabei direkt an die Silicagel-Schicht gebunden. Die Extraktionssäule wird mit 0,0 ml 70% Ethanol, 100 mM NaCl, 10 mM Na-Acetat pH 7,0 und mit 0,8 ml 90% Ethanol/Wasser gewaschen. Anschließend wird die von Salz befreite DNA in konzentrierter Form mit je 50 µl 1 mM Tris-HCl, 0,1 mM EDTA, pH 8,0 von der Silicagel-Schicht in eine weitere Mikrotiterplatte eluiert.

Die Herstellung der Zell-Lysate mit Hilfe der Zentrifugation ist ein langwieriges und aufwendiges Verfahren. Die Limitierung ist vor allem dann gegeben, wenn viele Proben routinemäßig präpariert werden müssen. Die Zentrifugation hat den Nachteil, daß sie sich nicht automatisieren läßt.

Ein weiterer Gegenstand (und Verfahren) der Erfindung ist eine Vorrichtung und ein Verfahren zur automatischen Durchführung des Verfahrens ohne Zentrifugation in Form einer Filtrationseinheit, die der eigentlichen Reinigung der Nukleinsäure vorgeschaltet ist.

Dabei wird die Probe nach bekannter Weise mit Proteinasen, Detergentien und/oder Temperatur oder Alkali lysiert. Dieses rohe Lysat wird direkt auf den Filtrationsaufsatz dekantiert, überführt oder pipettiert. Die Filterschicht des Filtrationsaufsatzes ist so aufgebaut, daß ein Verstopfen der Filter durch die Zelltrümmer, ausgefallene Proteine oder Detergentien vermieden wird. Das Zell-Lysat wird durch die Filterschicht mit einem Stempel oder Überdruck durchgedrückt oder unter Anlegen eines Vakuums durchgesaugt. Dabei werden alle ungelösten Bestandteile zurückgehalten und das klare Lysat tropft direkt auf die Adsorptionsschicht. Durch die Wahl der geeigneten Adsorptionsbedingungen wird die Nukleinsäure an der Adsorptionsschicht adsorbiert. Die Filtrationseinheit mit dem Filterkuchen wird von der Adsorptionseinheit abgetrennt und/oder verworfen und für die Analyse des Filterkuchens aufgehoben. Die Adsorptionseinheit wird mit geeigneten Lösungsmitteln oder Puffern nachgewaschen, um unerwünschte Bestandteile zu entfernen und die erwünschte Probe wird zum Schluß mit einem geeigneten Elutionsmittel eluiert.

Beispielsweise läßt sich nach dem erfindungsgemäßen Verfahren Plasmid DNA ohne eine Klar-Zentrifugation in einer Kühlzentrifuge präparieren. 96 x 1 ml Kulturen von Plasmid pBluescript in XL1 Blue E.coli Zellen, werden im 2 x YT Medium 18 Stunden bei 37 °C in einer Mikrotiterplatte mit 1,5 ml Vertiefungen (Fa. Beckmann, München) kultiviert. Die Zellen werden in einer Mikrotiterzentrifuge für 10 Minuten bei 2.500 g pelletiert.

Mit einer 8-Kanal Multichanel-Pipette (Fa. Matrix Technologies, Lowell, MA, USA) werden je 0,25 ml 50 mM Tris-HCl, 10 mM EDTA, 100 µg/ml RNAglA in die Mikrotitervertiefungen pipettiert und die Zellen 5 Minuten auf einem Vibrations-Schüttler resuspendiert. Die resuspendierten Zellen werden in das Probenreservoir des Filtrationsaufsatezs überführt und mit 0,25 ml 0,2 M NaOH/1% SDS versetzt. Die Probe wird 5 Minuten auf einen Vibrations-schüttler geschüttet oder mit einem Stopfen oder einer Klebefolie verschlossen und gemischt, oder durch mehrmaliges Auf- und Abpipettieren gemischt.

Nach 5 Minuten Inkubation bei Raumtemperatur zur Lyse wird zur Neutralisation der NaOH und Präzipitation des SDS 0,25 ml 3M K-Acetat, 2 M Essigsäure zugegeben und nach einem der oben beschriebenen Verfahren gemischt. Dieses rohe Zell-Lysat wird nun statt einer Zentrifugation auf einer Vakuumkammer bei 10 mbar - 800 mbar Vakuum durch die Filtrationsschicht gesaugt. Eine asymmetrische oder eine stufenweise Porosität im Bereich 200 µm bis 5 µm aufweisende Filterschicht mit einer Dicke von 2 - 10 mm hält die Zellbruchstücke und anderen ungelösten bzw. präzipitierten Bestandteile zurück ohne zu verstopfen. Das Plasmid DNA enthaltende, klare Zell-Lysat tropft durch die Filterschicht auf die Adsorptionsschicht (Anionenaustauscher oder Silicagel) und die DNA wird adsorbiert, wogegen Proteine, RNA und andere zelluläre Metabolite unter den gegebenen Salzbedingungen nicht binden. Die Filtration ist nach ca 10 bis 60 Sekunden beendet. Der Filteraufsatz wird abgenommen und zusammen mit dem Filterkuchen verworfen.

Die gebundene DNA wird mit 1 ml 1 M NaCl, 15% Ethanol, 50 mM Tris-HCl pH 7,0 und 2 mal mit 1 ml 1,5 M NaClO₄, 10 mM Na-Acetat, pH 6,5 gewaschen und mit 7 M NaClO₄, 15% Ethanol, 50 mM Tris-HCl, pH 7,0 von Anionenaustauscher eluiert und nach Passieren der Trennschicht aus einem Nylonnetz oder PP-Vlies unter den hohen Salzkonzentrationen sofort an die Silicagelschicht gebunden. Dabei binden die Proteine und RNA bei 1 M - 2 M NaClO₄ nicht an die Silicagelschicht und werden ausgewaschen. Die Silicagelschicht wird zum Entfernen der restlichen Spuren an Proteinen mit 1 ml 7 M Guanidin HCl, 10 mM Na-Acetat, pH 7,0 gewaschen. Die Hochsalzlösung an 7 M NaClO₄ wird zweckmäßigerweise mit 1 ml 70% EtOH, 100 mM NaCl, 10 mM Na-Acetat, pH 7,0 und 1 ml 90% Ethanol/Wasser oder 1 ml 90% Aceton/Wasser ausgewaschen. Nach dem Trocknen wir die Plasmid DNA salzfrei und inkonzentrierter Form mit 50 µl 1 mM Tris-HCl, 0,1 mM EDTA, pH 8,5 eluiert.

Auf diese Weise läßt sich die Plasmid DNA in kürzester Zeit ohne Zentrifugation, Phenol/Chloroform-Extraktion und ohne Alkoholfällung mit einer Ausbeute von 50% bis 80% inkonzentrierter Form isolieren. Bei der Verwendung einer beschriebenen Mikrotiterplattenversion lassen sich 96 Plasmid-Minipreps von 1 - 2 ml E.coli Kulturen mit einer Ausbeute von 1 - 10 µm DNA in ca. 60 Minuten präparieren von einer Person. Die bisher bekannten Verfahren benötigen dazu 6 bis 12 Stunden.

### Beispiel 6

Plasmid Miniprep mit einer Vorrichtung nach Figur 7 Eine 1,5 ml XL Blue E.coli Kultur mit pUC 18 Plasmid DNA in LB-Medium wird 5 Minuten bei 10.000 g zentrifugiert, um die Zellen zu pelletieren. Das Zell-Pellet wird in 0,25 ml 50 ml Tris-HCl, 10 mM EDTA, pH 8,0, 100 µg/ml RNAse A resuspendiert. Zur Zell-Lyse werden 0,25 ml 0,2 M NaOH, 1% SDS werden zur Zellsuspension gegeben, vorsichtig gemischt und 5 Minuten bei Raumtemperatur stehen gelassen. Danach wird 0,25 ml 3M K-Acetat, 2 M Essigsäure zur Neutralisation zugegeben, gemischt und 15 Minuten auf Eis inkubiert. Das Lysat wird in die Filtrationsvorrichtung nach Figur 7 überführt. Die ganze Vorrichtung wird auf eine Vakuum-Kammer aufgesetzt und das Zell-Lysat mit 20 mbar - 800 mbar durch die Vorrichtung gesaugt. Alternativ kann die Probe mit einem Kolbenstempel oder Uberdruck durch die Filtrationsschichten gedrückt werden. Nach der Filtration wird die Filtrationsvorrichtung abgenommen und der Filterkuchen mit den Zellbrucstücken, den denaturierten Proteinen und dem ausgefallenen SDS verworfen.

Die Extraktionssäule wird 2 mal mit 0,8 ml 1 M NaCl, 15% Ethanol, 50 mM MOPS, pH 7,0 gewaschen, um RNA und Proteine zu entfernen.Die DNA wird mit 1 ml 1,25 M NaCl, 15% Ethanol, 50 mM Tris-HCl, pH 8,5 eluiert. Die eluierte DNA wird zur Entsalzung und zur Konzentrierung mit Alkohol gefällt und das Alkohol-Pellet durch eine Zentrifugation pelletiert.

### Beispiel 7

### Präparation von Plasmid DNA mit einer Vorrichtung nach Figur 8

Eine 1,5 ml XL Blue E.coli Kultur mit pUC 18 Plasmid DNA in LB-Medium wird 5 Minuten bei 10.000 g zentrifugiert, um die Zellen zu pelletieren. Das Zell-Pellet wird in 0,25 ml 50 ml Tris-HCl, 10 mM EDTA, pH 8,0, 100 µg/ml RNAse A resuspendiert und in die Filtrationsvorrichtung überführt. Zur Zell-Lyse werden 0,25 ml 0,2 M NaOH, 1% SDS zur Zellsuspension in die Filtrationsvorrichtung nach Figur 8 gegeben, die Vorrichtung mit einem Stopfen oder einer Klebefolie verschlossen, vorsichtig gemischt und 5 Minuten bei Raumtemperatur stehen gelassen. Danach wird 0,25 ml 3 M K-Acetat, 2 M Essigsäure zur Neutralisation zugegeben, gemischt und 15 Minuten auf Eis inkubiert. Die ganze Vorrichtung wird auf eine Vakuum-Kammer aufgesetzt und das Zell-Lysat mit 20 mbar - 800 mbar durch die Vorrichtung gesaugt. Alternativ kann die Probe mit einem Kolbenstempel oder Überdruck durch die Filtrationsschichten gedrückt werden. Nach der Filtration wird die Filtrationsvorrichtung abgenommen und der Filterkuchen mit den Zellbruchstücken, den denaturierten Proteinen und dem ausgefallenen SDS verworfen. Die Extraktionssäule wird 2 mal mit 0,8 ml 1 M NaCl, 15% Ethanol, 50 mM MOPS, pH 7,0 gewaschen, um RNA und Proteine zu entfernen. Die DNA wird mit 1 ml 1,25 M NaCl, 15% Ethanol, 50 mM Tris-HCl, pH 8,5 eluiert. Die eluierte DNA wird zur Entsaltzung und zur Konzentrierung mit Alkohol gefällt und das Alkohol-Pellet durch eine Zentrifugation pelletiert.

### Beispiel 8

### Präparation von Plasmid DNA an einer Silicagel-Schicht mit einer Vorrichtung nach Figur 10

Eine 1,5 ml XL Blue E.coli Kultur mit pUC 18 Plasmid DNA in LB-Medium wird 5 Minuten bei 10.000 g zentrifugiert, um die Zellen zu pelletieren. Das Zell-Pellet wird in 0,25 ml 50 ml Tris-HCl, 10 m MEDTA, pH 8,0, 100 µg/ml RNAse A resuspendiert und in die Filtrationsvorrichtung nach Figur 10 überführt. Zur Zell-Lyse werden 0,25 ml 0,2 M NaOH, 1% SDS zur Zellsuspension in die Filtrationsvorrichtung gegeben, die Vorrichtung mit einem Stopfen oder einerKlebefolie verschlossen, vorsichtig gemischt und 5 Minuten bei Raumtemperatur stehen gelasen. Danach wird 0,5 ml 5,5 M Guanidin-HCl, 0,25 M K-Acetat, pH 5,5 zur Neutralisation zugegeben, gemischt und 15 Minuten auf Eis inkubiert. Die ganze Vorrichtung nach Abb. 10 wird auf eine Vakuum-Kammer aufgesetzt und das Zell-Lysat mit 20 mbar - 800 mbar durch die Vorrichtung gesaugt. Alternativ kann die Probe mit einem Kolbenstempfel oder Überdruck durch die Filtrationsschicht abgenommen und der Filterkuchen mit den Zellbruchstücken, den denaturierten Proteinen und dem ausgefallenen SDS verworfen. Die Extraktionssäule wird 2 mal mit 1 ml 7 M NaClO₄, 10 mM Na-Acetat, pH 7,0 gewaschen und mit 0,8 ml 90% Ethanol/Wasser gewaschen und die Ethanolspuren durchgesaugt. Zum Schluß wird die DNA mit 50 µl 10 mM Tris-HCl, 1 mM EDTA, pH 8,0 eluiert und in neuen 1,5 ml Röhrchen aufgefangen.

Die eluierte DNA kann direkt in einer enzymatischen Reaktion wie zum Beispiel Restriktionsspaltung, Markierung, Sequenzierung oder Amplifikation eingesetzt werden.

### Beispiel 9

### Präparation von 8 x Plasmid DNA in einem Mikrotiterstreifen

8 mal 1,5 ml XL Blue E.coli Kulturen mit pUC 18 Plasmid DNA in LB-Medium werden 5 Minuten bei 10.000 g zentrifugiert, um die Zellen zu pelletieren. Die Zell-Pellets werden in 0,25 ml 50 ml Tris-HCl,10 m MEDTA, pH 8,0, 100 µg/ml RNAse A resuspendiert und in die vorrichtung nach Figur 14 überführt. Zur Zell-Lyse werden 0,25 ml 0,2 M NaOH, 1% SDS zur Zellsuspension in die Filtrationsvorrichtung gegeben, die Vorrichtung mit einem Stopfen oder einer Klebefolie verschlossen, vorsichtig gemischt und 5 Minuten bei Raumtemperatur stehen gelassen. Danach wird 0,25 ml 3 M K-Acetat, 2 M Essigsäure zur Neutralisation zugegeben, gemischt und 15 Minuten auf Eis inkubiert. Die ganze Vorrichtung wird auf eine Vakuum-Kammer aufgesetzt und das Zell-Lysat mit 20mbar - 800 mbar durch die Vorrichtung esaugt. Alternativ kann die Probe mit Uberdruck durch die Filtrationsschichten gedrückt werden. Nach der Filtration wird die Filtrationsvorrichtung abgenommen und der Filterkuchen mit den Zellbruchstücken, den danaturierten Proteinen und dem ausgefallenen SDS verworfen. Die Extraktionssäule wird mit 0,8 ml 1 M NaCl, 15% Ethanol, 50 mM MOPS, pH 7,0 und mit 0,8 ml 1 M NaClO₄, 15% Ethanol, 10 mM Na-Acetat pH 7,0 gewaschen, um RNA und Proteine zu entfernen. Die DNA wird mit 7 M NAClO₄, 15% Ethanol, 10 mM Na-Acetat, pH 7,0 von der Anionenaustauscher-Schicht 10 eluiert und dabei direkt an die Silicagel-Schicht 11 gebunden. Die Extraktionssäule wird mit 0,8 ml 70% Ethanol, 100 mM NaCl, 10 mM Na-Acetat pH 7,0 und mit 0,8 ml 90% Ethanol/Wasser gewaschen. Die in der Extraktionsschicht vorhandenen Ethanol-H₂O-Reste werden durch ein Durchsaugen von Raumluft durch ein Vakuum für 1 - 2 Minuten verflüchtigt. Anschließend werden die 8 Proben mit je 50 µl 1 mM Tris-HCl, 0,1 mM EDTA, pH 8,0 eluiert.

### Beispiel 10

### Präparation von 8 x 1 ml M13 DNA mit einer Vorrichtung nach Figur 13

8 x 1 ml M13 Phagensuspension werden mit 0,5 ml 30% PEG 6000, 1,5 M NaCl versetzt und 10 Minuten auf Eis inkubiert. Die Proben werden auf eine Vorrichtung nach Abb. 13 überführt und das Phagenlysat wird auf einer Vakuum-kammer direkt durch eine Vorrichtung nach Figur 13 gesaugt und filtriert. Das Phagenpellet wird durch das Durchsaugen von 7M Guanidin-HCl, pH 7,0 lysiert und die DNA gleichzeitig an die Silicagelschicht 11 adsorbiert. Die Extraktionssäule wird 2 mal mit 1 ml 7 M Guanidin-HCl, 10 mM Na-Acetat, pH 7,0 gewaschen,um Proteine zu entfernen. Die Extraktionssäule wird mit 0,0 ml 70% Ethanol, 100 mM NaCl, 10 mM Na-Acetat pH 7,0 und mit 0,8 ml 70% Ethanol, 100 mM NaCl, 100 mM Na-Acetat pH 7,0 und mit 0,8 ml 90% Ethanol/Wasser gewaschen und für 1 - 2 Minuten Luft durchgesaugt. Zum Schluß wird die DNA mit 50 µl 10 mM Tris-HCl, 1 mM EDTA, pH 8,0 eluiert und in neuen 1,5 ml Röhrchen aufgefangen.

Die eluierte DNA kann direkt in einer enzymatischen Reaktion wie zum Beispiel Restriktionsspaltung, Markierung, Sequenzierung oder Amplifiation eingesetzt werden.

### Beispiel 11

### Präparation von 8 x 12 Plasmid DNA mit einer Vorrichtung nach Figur 14

96 mal 1,5 ml XL Blue E.coli Kulturen mit pUC 18 Plasmid DNA in LB-Medium werden 5 Minuten bei 2.500 g zentrifugiert, um die Zellen zu pelletieren. Die Zell-Pellets werden in 0,25 ml 50 ml Tris-HCl, 10 m MEDTA, pH 8,0, 100 µg/ml RNAse A resuspendiert und in die Vorrichtung mit einem Stopfen oder einer Klebefolie verschlossen, vorsichtig gemischt und 5 Minuten bei Raumtemperatur stehen gelassen. Danach wird 0,25 ml 3 M K-Acetat 2 M Essigsäure zur Neutralisation zugegeben, gemischt und 15 Minuten auf Eis inkubiert. Die ganze Vorrichtung wird auf eine Vakuum-Kammer aufgesetzt und das Zell-Lysat mit 20 mbar - 800 mbar durch die Vorrichtung gesaugt. Alternativ kann die Probe mit Überdruck durch die Filtrationsschichten gedrückt werden. Nach der Filtration wird die Filtrationsvorrichtung abgenommen und der Filterkuchen mit den Zellbruchstücken, den denaturierten Proteinen und dem ausgefallenen SDS verworfen. Die Extraktionssäule wird 0,8 ml 1 M NaCl, 15% Ethanol, 50 mM MOPS, pH 7,0 und mit 0,8 ml 1 M NaClO₄, 15% Ethanol, 10 mM Na-Acetat pH 7,0 gewaschen, um RNA und Proteine zu entfernen. Die DNA wird mit 7 M NaClO₄, 15% Ethanol, 10 mM Na-Acetat, pH 7,0 von der Anionenaustauscher-Schicht 10 eluiert und dabei direkt an die Silicagel-Schicht 11 gebunden. Die Extraktionssäule wird mit 0,8 ml 70% Ethanol, 100 mM NaCl, 10 mM Na-Acetat pH 7,0 und mit 0,8 ml 90% Ethanol/Wasser gewaschen.

Die in der Extraktionsschicht vorhandenen Ethanol-H₂O-Reste werden durch ein Durchsaugen von Raumluft durch ein Vakuum für 1 - 2 Minuten verflüchtigt. Anschließend werden die 96 Proben mit je 50 µl 1 mM Tris-HCl, 0,1 mM EDTA pH 8,0 eluiert und in neuen 1,5 ml Röhrchen aufgefangen. Die eluierte DNA kann direkt in einer enzymatischen Reaktion, wie zum Beispiel Restriktionsspaltung, Markierung, Sequenzierung oder Amplifikation eingesetzt werden.

### Beispiel 12

### Präparation von Plasmid-DNA ohne Konditionierung

Eine 3 ml Kultur in LB-Ampicillin-Medium mit pUC 18 transformierten HB 101 E. coli Zellen wird 10 Minuten bei 5.000 g zentrifugiert. Das Zellpellet wird in 0,25 ml 50 ml Tris-HCl, 10 m MEDTA, pH 8,0, 100 µg/ml RNAse A resuspendiert. Zur Zell-Lyse werden 0,25 ml 0,2 M NaOH, 1% SDS werden zur Zellsuspenison gegeben, vorsichtig gemischt und 5 Minuten bei Raumtemperatur stehen gelassen. Danach wird 0,25 ml 3 M K-Acetat, 2 M Essigsäure zur Neutralisation zugegeben, gemischt und 15 Minuten auf Eis inkubiert. Das Lysat wird 15 Minuten bei 10.000 g zentrifugiert und der Überstand vorsichtig abgehoben. Das klare Zell-Lysat wird auf eine DEAE-Anionenaustauscher-Extraktionssäule pipettiert und die Probe durch die Austauscherschicht durchgesaugt. Die Extraktionssäule wird mit 0,8 ml 1 M NaCl, 15% Ethanol, 50 mM MOPS, pH 7,0, 15% Ethanol, 10 mM Na-Acetat pH 7,0 gewaschen, um RNA und Proteine zu entfernen. Die DNA wird mit 0,7 ml 7 M NaClO₄, 15% Ethanol, 10 mM Na-Acetat, pH 7,0 auf eine Extraktionssäule mit einer Glasfasermembran gesaugt. Die eluierte DNA-Lösung in 7 M NaClO₄ wird durch die Glasfasermembran gesaugt und dabei direkt an die Silicagel-Schicht gebunden. Die Extraktionssäule wird mit 0,8 ml 70% Ethanol, 100 mM NaCl, 10 mM Na-Acetat pH 7,0 und mit 0,8 ml 90% Ethanol/ Wasser gewaschen. Spuren an EtOH werden eventuell durch ein Durchsaugen von Raumluft entfernt. Zum Schluß wird die DNA mit 10 µl 10 mM Tris-HCl, 1 mM EDTA, pH 8,0 eluiert und in neuen 1,5 ml Röhrchen aufgefangen.

### Beispiel 13

### Präparation von Plasmid-DNA mit Konditionierung

Eine 3 ml Kultur in LB-Ampicillin-Medium mit pUC 18 transformierten HB 101 E. coli Zellen wird 10 Minuten bei 5.000 g zentrifugiert. Das Zellpellet wird in 0,25 ml 50 ml Tris-HCl, 10 m MEDTA, pH 8,0, 100 µg/ml RNAse A resuspendiert. Zur Zell-Lyse werden 0,25 ml 1,2 M NaOH, 1% SDS werden zur Zellsuspension gegeben, vorsichtig gemischt und 5 Minuten bei Raumtemperatur stehen gelassen. Danach wird 0,25 ml 3 M K-Acetat, 2 M Essigsäure zur Neutralisation zugegeben, gemischt und 15 Minuten auf Eis inkubiert. Das Lysat wird 15 Minuten bei 10.000 g zentrifugiert und der Überstand vorsichtig abgehoben. Das klare Zell-Lysat wird auf eine DEAE-Anionenaustauscher-Extraktionssäule pipettiert und die Probe durch die Austauscherschicht durchgesaugt. Die Extraktionssäule wird mit 0,8 ml 1 M NaCl, 15% Ethanol, 50 mM MOPS, pH 7,0 gewaschen, um RNA und Proteine zu entfernen und mit 0,8 ml 1 M NaClO₄, 15 Ethanol, 10 mM Na-Acetat pH 5,0 konditioniert. Die DNA wird mit 0,7 ml 7 M NaClO₄, 15% Ethanol, 10 mM Na-Acetat, pH 7,0 auf eine Extraktionssäule mit einer Glasfasermembran gesaugt. Diese Glasfasermembran wurde vorher mit 0,2 ml 7 M NaClO₄, 15% Ethanol, 10 mM Na-Acetat, pH 7,0 konditioniert, um eine bessere Adsorption der DNA zu erreichen und Verluste in den ersten Tropfen zu vermeiden. Die eluierte DNA-Lösung in 7M NaClO₄ wird anschließend auf einer Vakuumvorrichtung durch die Glasfasermembran gesaugt und dabei direkt an die Silicagelschicht gebunden. Die Extraktionssäule wird mit 0,8 ml 70% Ethanol, 100 mM NaCl, 10 mM Na-Acetat pH .0 und mit 0,8 ml 90% Ethanol/ Wasser gewaschen. Spuren n EtOH werden eventuell durch ein Durchsaugen von Raumluft entfernt. Zum Schluß wird die DNA mit 100 µl 10 mM Tris-HCl, 1 mM EDT, pH 8,0 eluiert und in neuen 1,5 ml Rörhchen aufgefangen.

Die Vorkonditionierung kann auch durch eine mit 7 M NaClO₄, 15% Ethanol, 10 mM Na-Acetat, pH 7,0 getränkte und getrocknete Membran erreicht werden. Durch die Vorkonditionierung werden die Adsorptionsverluste von 30 % auf unter 5% reduziert und die Gesamtausbeute der DNA erhöht sich von 50 - 60% auf 80 - 90%.

## Patentansprüche

1. Verfahren zur Isolierung und Reinigung von Nukleinsäuren, insbesondere Plasmid- oder genomischer DNA, aus Zellen oder anderen Quellen, wobei
a) die Nukleinsäuren enthaltenden Zellen aufgeschlossen und die Zelltrümmer entfernt werden oder sonstige nukleinsäurehaltige Proben mit Anionenaustauschern behandelt werden, und zwar in Pufferlösungen mit geringer Ionenstärke,
b) danach die Nukleinsäuren mit einem Puffer hoher Ionenstärke von dem Anionenaustauscher desorbiert werden, um danach
c) im Puffer hoher Ionenstärke oder in Gegenwart von niederen Alkoholen, und/oder Polyethylenglykol mit einem mineralischen Trägermaterial behandelt zu werden unter Adsorption der Nukleinsäure an die Oberfläche der mineralischen Trägerstoffe, woraufhin
d) eine Desorption der Nukleinsäure mit Wasser oder einer Pufferlösung mit geringer Ionenstärke erfolgt.

2. Verfahren nach Anspruch 1, dadurch gekennzeichnet, daß die Verfahrensschritte b) und c) unmittelbar aufeinanderfolgend durchgeführt werden.

3. Verfahren nach Anspruch 1 und/oder 2, wobei Zentrifugations- oder Filtrationsschritte dem Schritt a) vorgeschaltet werden, um nicht gelöste Bestandteile mechanisch abzutrennen.

4. Verfahren nach mindestens einem der Ansprüche 1 bis 3, wobei zwischen den Schritten a) und b) ein oder mehrere Waschschritte mit Pufferlösungen mit geringer oder pro Waschschritt steigender Ionenstärke erfolgen.

5. Verfahren nach mindestens einem der Ansprüche 1 bis 4, wobei zwischen den Schritten c) und d) ein oder mehrere Waschschritte mit einer Pufferlösung hoher Ionenstärke erfolgen.

6. Verfahren nach mindestens einem der Ansprüche 1 bis 5, wobei zwischen den Schritten c) und d) mindestens ein Waschschritt mit wäßrig/alkoholischer Lösung erfolgt.

7. Verfahren nach mindestens einem der Ansprüche 1 bis 6, wobei zwischen den Schritten c) und d) des Anspruchs 1 ein Waschschritt mit etwas höherer Ionenstärke entsprechend einer 1,5 molaren Natriumperchlorat-Lösung bei einem pH-Wert von ca. 5 durchgeführt wird.

8. Verfahren nach mindestens einem der Ansprüche 1 bis 7, wobei ein Anionenaustauscher vorzugsweise mit hoher Oberflächenladung verwendet wird.

9. Verfahren nach mindestens einem der Ansprüche 1 bis 8, wobei die Nukleinsäure aus einer PCR- (Polymerase Chain Reaction), SSSR- (Self-Sustained- Sequence Replication), Ligase-Chain-Reaction stammt.

10. Verfahren nach mindestens einem der Ansprüche 1 bis 9, wobei die Nukleinsäure 10 Nukleotide bis 200.000 Nukleotide umfaßt.

11. Verfahren nach mindestens einem der Ansprüche 1 bis 10, wobei die Nukleinsäuren aus Bakterien, Zellkulturen, Blut, Gewebe, Urin, Viren oder anderen biologischen Quellen stammen.

12. Verfahren nach mindestens einem der Ansprüche 1 bis 11, wobei markierte Nukleinsäuren, insbesondere mit Biotin markierte Nukleinsäuren, fluoreszenz markierte Nukleinsäuren, wie mit Fluorescein-Iso-thiocyanat markierte oder radioaktiv markierte Nukleinsäuren eingesetzt werden.

13. Verfahren nach mindestens einem der Ansprüche 1 bis 12, wobei als mineralische Träger Silicagel, Glas, Zeolithe, Aluminiumoxid, Titandioxid, Zirconiumoxid, Kaolin und/oder Kieselalgen verwendet werden.

14. Verfahren nach mindestens einem der Ansprüche 1 bis 13, wobei poröse oder nicht poröse Matrices verwendet werden mit einer Partikelgröße von 1 µm bis 250 µm, vorzugsweise 10 bis 30 µm.

15. Verfahren nach mindestens einem der Ansprüche 1 bis 14, wobei eine Silicagelsuspension mit einer Partikelgröße von 1 bis 250 µm, vorzugsweise 1 bis 5 µm verwendet wird.

16. Verfahren nach mindestens einem der Ansprüche 1 bis 15, wobei der Anionenaustauscher eine Partikelgröße von 1 bis 250 µm, vorzugsweise 10 bis 100 µm, und einem Porendurchmesser von 1 bis 2.500 nm, vorzugsweise 100 bis 400 nm, aufweist.

17. Vorrichtung zur Isolierung und Reinigung von Nukleinsäuren mit einem Hohlkörper (1) mit einer Einlaßöffnung (7) und einer Auslaßöffnung (8), wobei im Hohlkörper (1) zwischen zwei Fixiereinrichtungen (5, 6) ein pulverförmiges erstes Material auf Silicagelbasis (10) angeordnet ist, dadurch gekennzeichnet, daß ein zweites Material (11) zwischen dem ersten Material (10) und der Auslaßöffnung (8) angeordnet ist, wobei die ersten und zweiten Materialien (10, 11) unterschiedliche Adsorptionscharakteristika für Nukleinsäuren aufweisen.

18. Vorrichtung nach Anspruch 17, dadurch gekennzeichnet, daß die Fixiereinrichtungen (5, 6) poröse Scheiben aus gesintertem Glas oder Keramik (Fritten) oder Membranen aus Kunststoffen, wie Polyethylen, Polypropylen, Polytetrafluorethylen oder Nylon, sind.

19. Vorrichtung nach Anspruch 17 und/oder 18, dadurch gekennzeichnet, daß die Materialien (10, 11) direkt aneinandergrenzen, und zwar in getrennten Schichten, und gemeinsam von den Fixiereinrichtungen (5, 6) gehalten werden.

20. Vorrichtung nach mindestens einem der Ansprüche 17 bis 19, dadurch gekennzeichnet, daß die Materialien (10, 11) durch eine Trenneinrichtung (13) getrennt sind.

21. Vorrichtung nach Anspruch 20, dadurch gekennzeichnet, daß die Trenneinrichtung (13) eine poröse Scheibe, vorzugsweise aus gesintertem Glas (Fritte), oder eine Kunststoffmembran, vorzugsweise aus Nylon, ist.

22. Vorrichtung nach mindestens einem der Ansprüche 17 bis 21, dadurch gekennzeichnet, daß das zweite Material (11) in dem einen Kanal bildenden Auslaß röhrchen (18), das einen geringeren Querschnitt als der Hohlkörper (1) aufweist, zwischen den Fixiereinrichtungen (5, 15) fixiert ist.

23. Vorrichtung nach Anspruch 22, wobei das zweite Material (11) vom ersten Material (10) nur durch eine gemeinsame Einrichtung (17) getrennt ist.

24. Vorrichtung nach mindestens einem der Ansprüche 17 bis 23, dadurch gekennzeichnet, daß das erste Material (10) aus einem Anionaustauscher auf Silicagelbasis besteht, während das zweite Material (11) aus einem Silicaglas besteht.

25. Vorrichtung nach mindestens einem der Ansprüche 17 bis 24, dadurch gekennzeichnet, daß die Materialien (10, 11) pulverförmig und/oder Preßkörper sind.

26. Vorrichtung nach mindestens einem der Ansprüche 17 bis 24, wobei die Partikel der Materialien (10, 11) in einem Trägernetz aus inerten Kunststoffen eingebettet sind.

27. Vorrichtung nach Anspruch 26, dadurch gekennzeichnet, daß das Trägernetz aus Teflon besteht.

28. Vorrichtung nach mindestens einem der Ansprüche 24 bis 27, dadurch gekennzeichnet, daß im Hohlkörper (1) eine weitere Schicht (12) zwischen dem Einlaß (7) und dem ersten Material (10) angeordnet ist, die als mechanische Filtereinrichtung wirkt.

29. Vorrichtung nach Anspruch 28, dadurch gekennzeichnet, daß die dritte Schicht (12) ein asymmetrischer Filter ist, wobei die Porengrößen des Filters in Fließrichtung abnimmt.

30. Vorrichtung nach einem der Ansprüche 28 und/oder 29, wobei der asymmetrische Filter aus gesintertem Glas mit abnehmender Porengröße oder übereinandergeschichteten Kunststoffmembranen mit abnehmender Porengröße besteht.

31. Verwendung der Vorrichtung nach einem der Ansprüche 17 bis 30 in einem Verfahren gemäß mindestens einem der Ansprüche 1 bis 15 zur Proteinentfernung aus einer nukleinsäurehaltigen Probe unter Vermeidung einer phenolischen, phenolisch/Chloroform oder Chloroformextraktion.

32. Verwendung eines Wasch- oder Adsorptionspuffers zur Durchführung des Verfahrens nach einem der Ansprüche 1 bis 16, dadurch gekennzeichnet, daß die Lösung 1 bis 7 M Natriumperchlorat, 1 bis 7 M Guanidinhydrochlorid, 1 bis 5 M Natriumchlorid, 1 bis 6 M Natriumiodid, 1 M Natriumchlorid/20% Ethanol oder niedere Alkohole wie Methanol, Ethanol, Propanol, Isopropanol, Butanol und/oder Polyethylenglykol enthält.

33. Verwendung eines Puffersystems zur Elution der adsorbierten Nukleinsäuren in einem Verfahren gemäß mindestens einem der Ansprüche 1 bis 16, wobei der Puffer Wasser, Tris bei einem pH-Wert von 5 bis 9 enthält.

34. Verwendung der gemäß einem der Verfahren 1 bis 16 gewonnen Nukleinsäuren in einer der folgenden enzymatischen Reaktionen, wie Restriktionsabdauung, Sequenzierung, Amplifikation, Markierung.

35. Verfahren zur Isolierung und Reinigung von Nukleinsäuren aus Zellen oder anderen Quellen, wobei
a) die Zelltrümmer oder sonstige Partikel durch eine Filterschicht mit, in Fließrichtung der Probe gesehen, abnehmender Porengröße entfernt werden,
b) wobei dann das Efluat mit einem Anionenaustauscher in Pufferlösungen mit geringer Ionenstärke behandelt wird.

36. Vorrichtung zur Durchführung des Verfahrens nach Anspruch 35, wobei mindestens eine Filterschicht (12, 20, 21 oder 22) im Lumen eines im wesentlichen zylindrischen Hohlkörpers (1) vor einer zwischen zwei Einrichtungen (5, 6) fixierten Schicht (10) mit Anionenaustauschereigenschaften, aus der Richtung der Einlaßöffnung (7) her gesehen, angeordnet ist.

37. Verfahren zur Isolierung und Reinigung von Nukleinsäuren aus Zellen oder anderen Quellen, wobei
a) die Zelltrümmer oder sonstige Partikel durch eine Filterschicht mit, in Fließrichtung der Proben gesehenen, abnehmenden Filterporengrößen entfernt werden,
wobei
b) das Effluat danach mit einem mineralischen Träger in Pufferlösungen hoher Ionenstärke behandelt wird.

38. Vorrichtung zur Durchführung des Verfahrens nach Anspruch 37, wobei mindestens eine Filterschicht (12, 20, 21 oder 22) im Lumen eines im wesentlichen zylindrischen Hohlkörpers (1) vor einer zwischen zwei Einrichtungen (5, 6) fixierten Schicht (11), die Nukleinsäuren bei hoher Ionenstärke der ent sprechenden Lösung zu binden vermag, aus der Richtung der Einlaßöffnung (7) her gesehen, angeordnet ist.

## Claims

1. A method for the isolation and purification of nucleic acids, especially plasmid or genomic DNA, from cells or other sources wherein
a) the cells containing the nucleic acids are lysed, and the cell debris removed, or other samples containing nucleic acids are treated with anion exchangers, in buffer solutions having a low ionic strength;
b) thereafter, the nucleic acids are desorbed from the anion exchanger with a buffer having a high ionic strength, and then
c) treated with a mineral support material in said buffer having a high ionic strength or in presence of lower alcohols and/or polyethylene glycol to adsorb the nucleic acid to the surface of the mineral support material, followed by
d) desorption of the nucleic acid with water or a buffer solution having a low ionic strength.

2. The method according to claim 1, characterized in that process steps b) and c) are performed in immediate succession.

3. The method according to claim 1 and/or 2, wherein centrifugation or filtration steps are interposed prior to step a) to separate non-dissolved components mechanically.

4. The method according to at least one of claims 1 to 3, wherein one or more washing steps with buffer solutions having low or increasing, from one washing step to the next, ionic strengths are performed between steps a) and b).

5. The method according to at least one of claims 1 to 4, wherein one or more washing steps with a buffer solution having a high ionic strength are performed between steps c) and d).

6. The method according to at least one of claims 1 to 5, wherein at least one washing step with an aqueous/alcoholic solution is performed between steps c) and d).

7. The method according to at least one of claims 1 to 6, wherein a washing step with somewhat higher ionic strength, corresponding to a 1.5 M sodium perchlorate solution, at a pH value of about 5 is performed between steps c) and d) of claim 1.

8. The method according to at least one of claims 1 to 7, wherein an anion exchanger having a high surface charge is preferably used.

9. The method according to at least one of claims 1 to 8, wherein said nucleic acid is derived from a PCR (polymerase chain reaction), SSSR (self-sustained sequence replication), or ligase chain reaction.

10. The method according to at least one of claims 1 to 9, wherein said nucleic acid comprises from 10 to 200,000 nucleotides.

11. The method according to at least one of claims 1 to 10, wherein said nucleic acids are derived from bacteria, cell cultures, blood, tissues, urine, viruses or other biological sources.

12. The method according to at least one of claims 1 to 11, wherein labeled nucleic acids, especially biotin-labeled nucleic acids, fluorescence-labeled nucleic acids, such as fluorescein isothiocyanate labeled nucleic acids, or radiolabeled nucleic acids are employed.

13. The method according to at least one of claims 1 to 12, wherein silica gel, glass, zeolites, alumina, titania, zirconia, china clay and/or diatoms are used as said mineral supports.

14. The method according to at least one of claims 1 to 13, wherein porous or non-porous matrixes having a particle size of from 1 µm to 250 µm, preferably from 10 to 30 µm, are used.

15. The method according to at least one of claims 1 to 14, wherein a silica gel suspension having a particle size of from 1 to 250 µm, preferably from 1 to 5 µm, is used.

16. The method according to at least one of claims 1 to 15, wherein said anion exchanger has a particle size of from 1 to 250 µm, preferably from 10 to 100 µm, and a pore diameter of from 1 to 2,500 nm, preferably from 100 to 400 nm.

17. A device for the isolation and purification of nucleic acids, comprising a hollow body (1) having an inlet (7) and an outlet (8), wherein a powdery first material based on silica gel (10) is disposed in said hollow body (1) between two fixing means (5, 6), characterized in that a second material (11) is disposed between said first material (10) and said outlet (8), wherein said first and second materials (10, 11) have different adsorption characteristics for nucleic acids.

18. The device according to claim 17, characterized in that said fixing means (5, 6) are porous disks of sintered glass or ceramics (frits) or plastic membranes, such as polyethylene, polypropylene, polytetrafluoroethylene or nylon.

19. The device according to claim 17 and/or 18, characterized in that said materials (10, 11) are immediately adjacent in separate layers and are commonly held in place by fixing means (5, 6).

20. The device according to at least one of claims 17 to 19, characterized in that said materials (10, 11) are separated by a separating means (13).

21. The device according to claim 20, characterized in that said separating means (13) is a porous disk, preferably of sintered glass (frit), or a plastic membrane, preferably nylon.

22. The device according to at least one of claims 17 to 21, characterized in that said second material (11) is fixed in an outlet tube (18) forming a channel and having a smaller cross-sectional area than that of hollow body (1), between said fixing means (5, 15).

23. The device according to claim 22, wherein said second material (11) is separated from said first material (10) only by one common means (17).

24. The device according to at least one of claims 17 to 23, characterized in that said first material (10) consists of an anion exchanger based on silica gel whereas said second material (11) consists of a silica glass.

25. The device according to at least one of claims 17 to 24, characterized in that said materials (10, 11) are powders and/or pressed pellets.

26. The device according to at least one of claims 17 to 24, wherein the particles of said materials (10, 11) are embedded in a supporting network of inert plastic materials.

27. The device according to claim 26, characterized in that said supporting network is made of Teflon.

28. The device according to at least one of claims 24 to 27, characterized in that another layer (12) which acts as a mechanical filtering means is disposed in hollow body (1) between inlet (7) and said first material (10).

29. The device according to claim 28, characterized in that said third layer (12) is an asymmetrical filter, the pore sizes of said filter decreasing in the direction of flow.

30. The device according to any of claims 28 and/or 29, wherein said asymmetrical filter consists of sintered glass with decreasing pore size or layered plastic membranes with decreasing pore sizes.

31. Use of the device according to any of claims 17 to 30 in a method according to at least one of claims 1 to 15 for removing proteins from a sample containing nucleic acids, avoiding extractions with phenol, phenol/chloroform, or chloroform.

32. Use of a washing or adsorption buffer for performing the method according to any of claims 1 to 16, characterized in that the solution contains from 1 to 7 M sodium perchlorate, from 1 to 7 M guanidine hydrochloride, from 1 to 5 M sodium chloride, from 1 to 6 M sodium iodide, 1 M sodium chloride/20% ethanol or lower alcohols, such as methanol, ethanol, propanol, isopropanol, butanol and/or polyethylene glycol.

33. Use of a buffer system for eluting the adsorbed nucleic acids in a method according to at least one of claims 1 to 16, said buffer containing water, Tris at a pH value of from 5 to 9.

34. Use of the nucleic acids obtained by the method according to any of claims 1 to 16 in one of the following enzymatic reactions, such as restriction digestion, sequencing, amplification, labeling.

35. A method for the isolation and purification of nucleic acids from cells or other sources wherein
a) the cell debris or other particles are removed by a filter layer with decreasing pore size as seen in the direction of sample flow;
b) the effluent then being treated with an anion exchanger in buffer solutions having low ionic strengths.

36. A device for performing the method according to claim 35, in which at least one filter layer (12, 20, 21 or 22) is disposed in the lumen of an essentially cylindrical hollow body (1) with inlet (7), upstream from a layer (10) having anion exchange properties which is fixed between two means (5, 6).

37. A method for the isolation and purification of nucleic acids from cells or other sources wherein
a) the cell debris or other particles are removed by a filter layer with decreasing filter pore sizes as seen in the direction of sample flow;
b) the effluent then being treated with a mineral support in buffer solutions having high ionic strengths.

38. A device for performing the method according to claim 37, in which at least one filter layer (12, 20, 21 or 22) is disposed in the lumen of an essentially cylindrical hollow body (1) with inlet (7), upstream from a layer (11) capable of binding nucleic acids at a high ionic strength of the corresponding solution which is fixed between two means (5, 6).

## Revendications

1. Procédé pour isoler et purifier des acides nucléiques, en particulier un ADN plasmidique ou génomique, à partir de cellules ou d'autres sources, dans lequel :
a) les cellules contenant des acides nucléiques sont attaquées, et les débris cellulaires sont éliminés, ou bien d'autres échantillons contenant des acides nucléiques sont traités par des échangeurs d'anions, et plus précisément dans des solutions tampon ayant une faible force ionique,
b) puis les acides nucléiques sont désorbés de l'échangeur d'anions à l'aide d'un tampon ayant une force ionique plus grande, pour ensuite,
c) dans le tampon ayant une grande force ionique ou en présence d'alcools inférieurs et/ou de polyéthylèneglycol, être traités avec un matériau support minéral, avec adsorption de l'acide nucléique par la surface des matériaux supports minéraux, ce après quoi
d) une désorption de l'acide nucléique avec de l'eau ou une solution tampon ayant une faible force ionique est effectuée.

2. Procédé selon la revendication 1, caractérisé en ce que les étapes b) et c) sont mises en oeuvre immédiatement l'une après l'autre.

3. Procédé selon la revendication 1 et/ou 2, dans lequel des étapes de centrifugation ou de filtration sont mises en oeuvre avant l'étape a) pour séparer par voie mécanique les constituants non-dissous.

4. Procédé selon au moins l'une des revendications 1 à 3, dans lequel une ou plusieurs étapes de lavage, utilisant des solutions tampons ayant une faible force ionique ou une force ionique croissant à chaque étape de lavage, sont mises en oeuvre entre les étapes a) et b).

5. Procédé selon au moins l'une des revendications 1 à 4, dans lequel une ou plusieurs étapes de lavage avec une solution tampon ayant une grande force ionique sont mises en oeuvre entre les étapes c) et d).

6. Procédé selon au moins l'une des revendications 1 à 5, dans lequel au moins une étape de lavage par une solution aqueuse/alcoolique est mise en oeuvre entre les étapes c) et d).

7. Procédé selon au moins l'une des revendications 1 à 6, dans lequel une étape de lavage avec une force ionique un peu plus grande, correspondant à une solution de perchlorate de sodium 1,5 M à pH d'environ 5, est mise en oeuvre entre les étapes c) et d).

8. Procédé selon au moins l'une des revendications 1 à 7, dans lequel on utilise un échangeur d'anions, de préférence ayant une grande charge superficielle.

9. Procédé selon au moins l'une des revendications 1 à 8, dans lequel l'acide nucléique provient d'une PCR (amplification en chaîne par polymérase), d'une SSSR (réplication des séquences auto-entretenue), ou d'une réaction en chaîne par ligase.

10. Procédé selon au moins l'une des revendications 1 à 9, dans lequel l'acide nucléique comprend de 10 à 200 000 nucléotides.

11. Procédé selon au moins l'une des revendications 1 à 10, dans lequel les acides nucléiques proviennent de bactéries, de cultures cellulaires, de sang, de tissus, d'urine, de virus ou d'autres sources biologiques.

12. Procédé selon au moins l'une des revendications 1 à 11, dans lequel on utilise des acides nucléiques marqués, en particulier des acides nucléiques marqués à la biotine, des acides nucléiques marqués par fluorescence, tels que des acides nucléiques marqués par de l'isothiocyanate de fluorescéine, ou des acides nucléiques radiomarqués.

13. Procédé selon au moins l'une des revendications 1 à 12, dans lequel on utilise en tant que support minéral du gel de silice, du verre, des zéolites, de l'oxyde d'aluminium, du dioxyde de titane, de l'oxyde de zirconium, du kaolin et/ou de la terre de diatomées.

14. Procédé selon au moins l'une des revendications 1 à 13, dans lequel on utilise des matrices poreuses ou non-poreuses ayant une granulométrie de 1 à 250 µm et de préférence de 10 à 30 µm.

15. Procédé selon au moins l'une des revendications 1 à 14, dans lequel on utilise une suspension de gel de silice ayant une granulométrie de 1 à 250 µm et de préférence de 1 à 5 µm.

16. Procédé selon au moins l'une des revendications 1 à 15, dans lequel l'échangeur d'anions a une granulométrie de 1 à 250 µm et de préférence de 10 à 100 µm et un diamètre des pores de 1 à 2500 nm et de préférence de 100 à 400 nm.

17. Appareil pour isoler et purifier des acides nucléiques, comportant un corps creux (1), lequel comporte un orifice d'entrée (7) et un orifice de sortie (8), un premier matériau pulvérulent à base de gel de silice (10) étant disposé dans le corps creux entre deux dispositifs de fixation (5, 6), caractérisé en ce qu'un deuxième matériau (11) est disposé entre le premier matériau (10) et l'orifice de sortie (8) , le premier et le deuxième matériaux (10, 11) présentant des caractéristiques différentes d'adsorption des acides nucléiques.

18. Appareil selon la revendication 17, caractérisé en ce que les dispositifs de fixation (5, 6) sont des disques poreux en verre ou en céramique fritté (frittes) ou des membranes en matériaux plastiques tels que le polyéthylène, le polypropylène, le polytétrafluoréthylène ou le Nylon.

19. Appareil selon la revendication 17 et/ou 18, caractérisé en ce que les matériaux (10, 11) sont contigus l'un à l'autre, plus précisément dans des couches distinctes, et sont retenus ensemble par les dispositifs de fixation (5, 6).

20. Appareil selon au moins l'une des revendications 17 à 19, caractérisé en ce que les matériaux (10, 11) sont séparés par un dispositif de séparation (13).

21. Appareil selon la revendication 20, caractérisé en ce que le dispositif de séparation (13) est un disque poreux, de préférence en verre fritté (fritte) ou une membrane plastique, de préférence en Nylon.

22. Appareil selon au moins l'une des revendications 17 à 21, caractérisé en ce que le deuxième matériau (11) est fixé entre les dispositifs de fixation (5, 15), dans un tube de sortie (18), formant un conduit et ayant une section transversale plus petite que le corps creux (1).

23. Appareil selon la revendication 22, dans lequel le deuxième matériau (11) n'est séparé du premier matériau (10) que par un dispositif commun (17).

24. Appareil selon au moins l'une des revendications 17 à 23, caractérisé en ce que le premier matériau (10) est constitué d'un échangeur d'anions à base de gel de silice, tandis que le deuxième matériau (11) est constitué d'un verre de silice.

25. Appareil selon au moins l'une des revendications 17 à 24, caractérisé en ce que les matériaux (10, 11) se présentent sous forme d'une poudre et/ou d'un comprimé.

26. Appareil selon au moins l'une des revendications 17 à 24, dans lequel les particules des matériaux (10, 11) sont incorporées dans un support réticulaire en plastiques inertes.

27. Appareil selon la revendication 26, caractérisé en ce que le support réticulaire est en Téflon.

28. Appareil selon au moins l'une des revendications 24 à 27, caractérisé en ce qu'une couche additionnelle (12) est disposée dans le corps creux (1) entre l'entrée (7) et le premier matériau (10), couche qui joue le rôle d'un dispositif de filtration mécanique.

29. Appareil selon la revendication 28, caractérisé en ce que la troisième couche (12) est un filtre asymétrique, dans lequel le diamètre des pores du filtre diminue dans le sens de l'écoulement.

30. Appareil selon l'une des revendications 28 et/ou 29, dans lequel le filtre asymétrique est constitué d'un verre fritté ayant un diamètre des pores décroissant, ou encore de membranes plastiques, disposées les unes au-dessus des autres, ayant un diamètre des pores décroissant.

31. Utilisation de l'appareil selon l'une des revendications 17 à 30, dans un procédé selon au moins l'une des revendications 1 à 15, pour éliminer les protéines d'un échantillon contenant des acides nucléiques, en évitant une extraction au phénol, au phénol/chloroforme ou au chloroforme.

32. Utilisation d'un tampon de lavage ou d'adsorption pour mettre en oeuvre le procédé selon l'une des revendication 1 à 16, caractérisé en ce que la solution contient du perchlorate de sodium 1 à 7 M, du chlorhydrate de guanidine 1 à 7 M, du chlorure de sodium 1 à 5 M, de l'iodure de sodium 1 à 6 M, du chlorure de sodium 1 M/de l'éthanol à 20 % ou d'autres alcools tels que le méthanol, l'éthanol, le propanol, l'isopropanol, le butanol et/ou le polyéthylèneglycol.

33. Utilisation d'un système tampon pour éluer les acides nucléiques adsorbés, dans un procédé selon au moins l'une des revendications 1 à 16, dans laquelle le tampon contient de l'eau, et du Tris à un pH de 5 à 9.

34. Utilisation des acides nucléiques obtenus par l'un des procédés 1 à 16 dans l'une des réactions enzymatiques suivantes, à savoir une digestion par des enzymes de restriction, un séquençage, une amplification, un marquage.

35. Procédé pour isoler et purifier des acides nucléiques à partir de cellules ou d'autres sources, dans lequel
a) les débris cellulaires ou d'autres particules sont isolés grâce à une couche filtrante dont les pores ont un diamètre qui diminue quand on regarde dans le sens d'écoulement de l'échantillon,
b) dans lequel l'effluent est ensuite traité avec un échangeur d'anions dans des solutions tampons ayant une faible force ionique.

36. Appareil pour mettre en oeuvre le procédé selon la revendication 35, dans lequel au moins une couche filtrante (12, 20, 21 ou 22) est disposée dans la lumière d'un corps creux (1), essentiellement cylindrique, et qui quand on regarde dans la direction de l'orifice d'entrée (7), se trouve en avant d'une couche (10), fixée entre deux dispositifs (5, 6) et présentant des propriétés d'échange d'anions.

37. Procédé pour isoler et purifier des acides nucléiques à partir de cellules et d'autres sources, dans lequel
a) les débris cellulaires ou d'autres particules sont isolés par une couche filtrante dont les pores filtrants ont un diamètre qui diminue quand on regarde dans le sens d'écoulement des échantillons,
et où
b) l'effluent est ensuite traité par un support minéral dans des solutions tampon ayant une force ionique plus grande.

38. Appareil pour mettre en oeuvre le procédé selon la revendication 37, dans lequel au moins une couche filtrante (12, 20, 21 ou 22) est fixée dans la lumière d'un corps creux (1) essentiellement cylindrique, et, quand on regarde dans la direction de l'orifice d'entrée (7), en avant d'une couche (11) , fixée entre deux dispositifs (5, 6), et susceptible de fixer les acides nucléiques quand la solution correspondante a une grande force ionique.
